# EUROPEAN PATENT APPLICATION

(11) **EP 4 660 308 A1**
(43) Date of publication of application: **10.12.2025**
(21) Application number: 24750308.9
(22) Date of filing: 30.01.2024
(51) Int. Cl.: C12N 15/34, C12N 5/10, C12N 7/01, C12N 15/38, C12N 15/54, C12N 15/55, C12N 15/63, C12N 15/864, C12N 15/90

(54) **HELPER GENE**

(30) Priority: 31.01.2023 JP 2023013329
(71) Applicant: Synplogen Co., Ltd., Kobe-shi, Hyogo 650-0047 (JP)
(72) Inventor: NISHIMURA, Yuya, Kobe-shi, Hyogo 650-0047 (JP); SAITO, Shunsuke, Kobe-shi, Hyogo 650-0047 (JP)
(74) Representative: Engelhard, Markus
(86) International application number: PCT/JP2024/002944
(87) International publication number: WO 2024/162359

(57) **Abstract**

The present disclosure provides a virus-derived gene that can be used for producing a novel virus-derived construct. More particularly, the virus-derived gene of the present disclosure includes a combination of an adenovirus-derived gene and a herpesvirus-derived gene. In one embodiment, the herpesvirus-derived gene is a gene associated with replication of a gene. In one embodiment, the herpesvirus-derived gene encodes a protein selected from a helicase, a primase, an ssDNA-binding protein (DBP), a DNA polymerase, and a DNA replication protein. In one embodiment, the herpesvirus-derived gene encodes a protein selected from an ssDNA-binding protein (DBP) and a DNA polymerase. In one embodiment, the herpesvirus-derived gene includes a gene selected from UL44, UL54, and UL57.

## Description

### Technical Field

The present disclosure provides a virus-derived gene useful for producing viral vectors. The present disclosure also provides a method using the virus-derived gene and a product obtained thereby.

### Background Art

With the development of gene therapeutics, various viral vectors such as adeno-associated virus (AAV) vectors have been used (Patent Literature 1). AAV vectors are often produced by adding adenovirus (AdV) as a helper virus to animal cells. There is a need for means for improving AAV vector production, such as increasing the yield of AAV vectors.

### Citation List

### Patent Literature

Patent Literature 1: WO 2001/090392 A

### Summary of Invention

### Solution to Problem

The present inventors have found that specific virus-derived genes exhibit advantageous effects in the production of AAV vectors, such as increasing the yield of AAV vectors. Based on this finding, the present disclosure provides specific virus-derived genes (alone or in combination). Similarly, the present disclosure provides nucleic acids (singular or plural) containing a specific virus-derived gene and use thereof. In addition, the present disclosure also provides producer cells and virus-derived constructs produced therefrom.

Accordingly, the present disclosure provides the following.

### (Item 1)

A nucleic acid containing an adenovirus-derived gene and a herpesvirus-derived gene.

### (Item 2)

The nucleic acid according to any one of the preceding items, in which the herpesvirus-derived gene is a gene associated with replication of a gene.

### (Item 3)

The nucleic acid according to any one of the preceding items, in which the herpesvirus-derived gene encodes a protein selected from a helicase, a primase, an ssDNA-binding protein (DBP), a DNA polymerase, and a DNA replication protein.

### (Item 4)

The nucleic acid according to any one of the preceding items, in which the herpesvirus-derived gene includes a gene selected from UL8, UL5, UL52, UL29 (ICP8), UL30, UL42, UL9, ORF6, ORF16, ORF28, ORF29, ORF55, BMRF1, BSLF1, BBLF4, BBLF2/BBLF3, BALF5, BALF2, UL102, UL105, UL70, UL57, UL54, UL44, UL112, U27, U38, U41, U79, U43, U73, U74, U77, U79, ORF9, and ORF59.

### (Item 5)

The nucleic acid according to any one of the preceding items, in which the herpesvirus-derived gene is selected from a DNA strand synthesis gene and a helicase-primase gene.

### (Item 6)

The nucleic acid according to any one of the preceding items, in which the herpesvirus-derived gene includes a gene selected from UL8, UL5, UL52, UL29 (ICP8), UL30, UL42, UL102, UL105, UL70, UL57, UL54, UL44, and UL112.

### (Item 7)

The nucleic acid according to any one of the preceding items, in which the herpesvirus-derived gene includes a DNA strand synthesis gene.

### (Item 8)

The nucleic acid according to any one of the preceding items, in which the herpesvirus-derived gene includes a gene selected from UL29 (ICP8), UL30, UL42, UL57, UL54, and UL44.

### (Item 9)

The nucleic acid according to any one of the preceding items, in which the herpesvirus-derived gene includes only one of a DNA strand synthesis gene or a helicase-primase gene.

### (Item 10)

The nucleic acid according to any one of the preceding items, in which the adenovirus-derived gene includes a helper gene.

### (Item 11)

The nucleic acid according to any one of the preceding items, in which the adenovirus-derived gene includes a gene selected from E1A, E1B, E2, E4, and VA.

### (Item 12)

The nucleic acid according to any one of the preceding items, in which the adenovirus is selected from serotypes 1 to 52.

### (Item 13)

The nucleic acid according to any one of the preceding items, in which the herpesvirus is selected from HHV-1 to HHV-8.

### (Item 14)

A composition for producing a viral vector, the composition containing the nucleic acid according to any one of the preceding items.

### (Item 15)

A composition for producing a virus-derived construct, the composition containing a nucleic acid containing a gene associated with replication of a virus-derived gene.

### (Item 16)

The composition according to any one of the preceding items, in which the gene associated with replication of the gene is derived from an adenovirus or a herpesvirus.

### (Item 17)

The composition according to any one of the preceding items, in which the gene associated with replication of the gene is derived from a herpesvirus.

### (Item 18)

The composition according to any one of the preceding items, in which the gene associated with replication of the gene includes a gene selected from UL8, UL5, UL52, UL29 (ICP8), UL30, UL42, UL9, ORF6, ORF16, ORF28, ORF29, ORF55, BMRF1, BSLF1, BBLF4, BBLF2/BBLF3, BALF5, BALF2, UL102, UL105, UL70, UL57, UL54, UL44, UL112, U27, U38, U41, U79, U43, U73, U74, U77, U79, ORF9, and ORF59.

### (Item 19)

The composition according to any one of the preceding items, in which the gene associated with replication of the gene is selected from a DNA strand synthesis gene and a helicase-primase gene.

### (Item 20)

The composition according to any one of the preceding items, in which the gene associated with replication of the gene includes a gene selected from UL8, UL5, UL52, UL29 (ICP8), UL30, UL42, UL102, UL105, UL70, UL57, UL54, UL44, and UL112.

### (Item 21)

The composition according to any one of the preceding items, in which the gene associated with replication of the gene includes a DNA strand synthesis gene.

### (Item 22)

The composition according to any one of the preceding items, in which the gene associated with replication of the gene includes a gene selected from UL29 (ICP8), UL30, UL42, UL57, UL54, and UL44.

### (Item 23)

The composition according to any one of the preceding items, in which the gene associated with replication of the gene includes only one of a DNA strand synthesis gene or a helicase-primase gene.

### (Item 24)

The composition according to any one of the preceding items, in which the gene associated with replication of the gene is derived from a herpesvirus selected from HHV-1 to HHV-8.

### (Item 25)

The composition according to any one of the preceding items, in which the gene associated with replication of the gene is derived from an adenovirus selected from serotypes 1 to 52.

### (Item 26)

The composition according to any one of the preceding items, in which the composition is for producing an adeno-associated virus (AAV) vector.

### (Item 27)

The nucleic acid according to any one of the preceding items, in which the nucleic acid is a plasmid.

### (Item 28)

The nucleic acid according to any one of the preceding items, in which the nucleic acid forms a complex with a cationic substance.

### (Item 29)

A method for producing a producer cell that produces a virus-derived construct, the method including a step of introducing the nucleic acid according to any one of the preceding items into a producer cell.

### (Item 30)

The method according to any one of the preceding items, in which at least a part of the nucleic acid is integrated into a chromosome of the producer cell.

### (Item 31)

A producer cell produced by the method according to any one of the preceding items.

### (Item 32)

A producer cell containing the nucleic acid according to any one of the preceding items.

### (Item 33)

A method for producing a virus-derived construct, the method including:
a step of introducing the nucleic acid according to any one of the preceding items into a producer cell; and
a step of forming a virus-derived construct in the producer cell.

### (Item 34)

A virus-derived construct produced by the method according to any one of the preceding items.

### (Item 35)

A virus-derived construct containing the nucleic acid according to any one of the preceding items.

In the present disclosure, it is intended that the one or the plurality of features may be provided in further combination in addition to the specified combination. Still further embodiments and advantages of the present disclosure will be appreciated by those skilled in the art upon reading and understanding the following detailed description, if necessary.

### Advantageous Effects of Invention

According to the present disclosure, it is possible to produce a virus-derived construct by a novel virus-derived gene, and it is possible to achieve high-yield production of the virus-derived construct.

### Brief Description of Drawings

[FIG. 1] FIG. 1 shows the yield of AAV vectors under each condition. The yield of AAV vectors is shown as the number of genome copies per 1 mL of cell culture supernatant.
[FIG. 2] FIG. 2 shows the yield of AAV vectors under each condition. The yield of AAV vectors is shown as the number of genome copies per 1 mL of cell culture supernatant.
[FIG. 3] FIG. 3 shows the yield of AAV vectors under each condition. The yield of AAV vectors is shown as the number of genome copies per 1 mL of cell culture supernatant.
[FIG. 4A] FIG. 4A shows the yield of AAV vectors under each condition. The yield of AAV vectors is shown as the number of genome copies per 1 mL of cell culture supernatant.
[FIG. 4B] FIG. 4B shows the yield of AAV vectors under each condition. The yield of AAV vectors is shown as the number of genome copies per 1 mL of cell culture supernatant.
[FIG. 5] FIG. 5 shows the results of Example 5. The titer of AAV3 is shown for each type of UL. It is shown that, when a gene associated with replication of HHV is added alone to the Ad helper genes, the yield of rAAV3 is increased for all such genes, as compared to the case where it is not added. Description of Embodiments

Hereinafter, the present disclosure will be described while showing the best mode. Throughout the present specification, the expression of singular forms is to be understood as including the concept of plural forms unless otherwise stated. Therefore, the singular article (for example, "a", "an", "the", or the like in English) should be understood to include the concept of plural forms unless otherwise stated. In addition, the terms used in the present specification are to be understood as being used in the sense commonly used in the art unless otherwise stated. Therefore, unless defined otherwise, all technical terms and scientific terms used in the present specification have the same meaning as commonly understood by those skilled in the art to which the present disclosure pertains. In the case of conflict, the present specification (including definitions) will control.

Hereinafter, the definitions of the terms and/or basic technical concepts that are particularly used in the present specification will be described as appropriate.

In the present specification, the terms "polynucleotide" and "nucleic acid" are used to have the same meaning, and refer to a polymer of nucleotides of any length. In the present specification, unless otherwise specified, the term "nucleic acid" refers to both a single nucleic acid molecule and a plurality of nucleic acid molecules, as well as both a single-stranded nucleic acid and a double-stranded nucleic acid. In the present specification, unless otherwise specified, a description stating that a nucleic acid includes a plurality of elements encompasses embodiments in which the plurality of elements are present on the same nucleic acid molecule and embodiments in which the plurality of elements are present on separate nucleic acid molecules, and encompasses embodiments in which the plurality of elements are present on the same double-stranded nucleic acid complex and embodiments in which the plurality of elements are present on separate double-stranded nucleic acid complexes. Examples of the nucleic acid include DNA, RNA, cDNA, mRNA, rRNA, tRNA, microRNA (miRNA), and lncRNA. The term also includes a "polynucleotide derivative". The term "polynucleotide derivative" refers to a polynucleotide containing a nucleotide derivative or having an unusual bond between nucleotides. The term "nucleotide derivative" refers to a nucleotide having a structure different from a normal nucleotide used in natural DNA or RNA, and examples thereof include a locked nucleic acid (LNA), an ethylene nucleic acid such as a 2'-O,4'-C-ethylene bridged nucleic acid (ENA), other bridged nucleic acids (BNAs), a hexitol nucleic acid (HNA), an amido-bridged nucleic acid (AmNA), a morpholino nucleic acid, tricyclo-DNA (tcDNA), a polyether nucleic acid (see, for example, US 5,908,845), and a cyclohexene nucleic acid (CeNA). Examples of the unusual bond between nucleotides include a bond between oligonucleotides in which a phosphate diester bond is converted into a phosphorothioate bond, a bond between oligonucleotides in which a phosphate diester bond is converted into an N3'-P5' phosphoramidate bond, and a bond between oligonucleotides in which ribose and a phosphate diester bond are converted to peptide nucleic acid bonds.

Unless otherwise indicated, a particular nucleic acid sequence is also intended to encompass a conservatively modified variant (for example, condensed codon substituent) and complementary sequence thereof, as well as the sequence explicitly indicated. Specifically, the condensed codon substituent may be achieved by generating a sequence in which the third position of one or more selected (or all) codons is substituted with a mixed base and/or deoxyinosine residue. Examples of variants based on specific wild-type sequences such as adenovirus serotypes 1 to 52 and adeno-associated virus serotypes 1 to 12 include not only known variants (for example, rh10, DJ, DJ/8, PHP.eB, PHP.S, AAV2-retro, AAV2-QuadYF, AAV2.7m8, AAV6.2, rh.74, AAV2.5, AAV-TT, Anc80, and the like) but also nucleic acids having sequences having at least 70%, at least 80%, at least 85%, at least 90%, at least 95%, at least 96%, at least 97%, at least 98%, at least 99%, or at least 99.5% sequence identity to the original sequence.

In the present specification, the term "gene" refers to a nucleic acid segment that performs a certain biological function. The biological function includes coding for polypeptides or proteins, coding for protein non-coding functional RNAs (rRNA, tRNA, microRNA (miRNA), lncRNA, and the like), regulating the production of polypeptides, proteins, or non-coding functional RNAs, specifically binding to specific proteins, and regulating cleavage or replication of nucleic acids. Therefore, in the present specification, the gene contains a transcriptional and translational regulatory sequence such as a promoter, a terminator, an enhancer, an insulator, a silencer, an origin of replication, or an internal ribosome entry site, and a nucleic acid segment required for packaging into viral particles, in addition to a nucleic acid segment encoding a protein or a protein non-coding functional RNA. In the present specification, the term "gene product" refers to an mRNA transcribed from a gene, a polypeptide or protein encoded by a gene, or a non-protein-coding functional RNA. In the present specification, the term "expression of a gene" refers to the production of a gene product. In particular, viral genes often encode multiple types of proteins of different lengths in a single nucleic acid region. In the present specification, when at least one type of protein that may be produced from a gene is produced, the gene is referred to as being expressed.

In the present specification, the term "adenovirus" (Ad) refers to a virus of the genus Mastadenovirus in the family Adenoviridae, and the viral particle contains a nucleocapsid and a linear double-stranded DNA genome, and has an icosahedral structure having a size of 90 to 100 nm. Infection by adenovirus is initiated by adsorption of the viral capsid to the coxackie-adenovirus receptor (CAR) on the cell surface, and then the virus may enter into the cell via the integrin on the cell surface. Thereafter, the viral genome that has escaped from the lysosome may reach the nucleus and result in replication of the viral genome. Viral replication is initiated by first expressing E1A protein and activating the expression of other early proteins E1B, E2, E3, and E4. Replication of the viral genome is initiated by formation of a complex in which a terminal protein (TP) expressed from E2 is covalently bound to deoxycytidine, and a polymerase is further bound thereto. The genome also encodes structural proteins including penton (L2), hexon (L3), scaffold protein (L4), and fiber protein (L5), and further includes five late transcription units (L1, L2, L3, L4, and L5) under the control of a single promoter. Both ends of the genome contain inverted terminal repeats (ITRs) required for virus replication. Structural proteins of the virus are translated in the cytoplasm and then translocate into the nucleus to constitute viral particles, recognize the packaging signal (ψ) of the viral genome, and package the genome. In addition, adenoviruses also produce non-protein-coding VA RNA, which is encoded by the VA gene. The adenoviral particles may have L2 and L3 capsids. To date, 52 human adenovirus serotypes have been identified and are classified into six subgroups based on hemagglutination properties and sequence homology: subgroup A (for example, serotypes 12, 18, and 31), subgroup B (for example, serotypes 3, 7, 11, 14, 16, 21, 34, 35, and 50), subgroup C (for example, serotypes 1, 2, 5, and 6), subgroup D (for example, serotypes 8, 9, 10, 13, 15, 17, 19, 20, 22 to 30, 32, 33, 36 to 39, and 42 to 48), subgroup E (for example, serotype 4), subgroup F (for example, serotypes 40 and 41), and a group of unclassified serotypes (for example, serotypes 49 and 51).

In the present specification, the term "herpesvirus" refers to human herpesviruses, which are viruses belonging to the family Herpesviridae that infect humans, and are DNA viruses having a linear double-stranded DNA genome, and the virion is a spherical particle with a diameter of 120 to 200 nm, in which an icosahedral capsid is enclosed by an envelope. In particular, examples of the herpesviruses described in the present specification include Human herpesvirus 1 (HHV-1) (also referred to as herpes simplex virus type 1 (HSV-1)) and Human herpesvirus 2 (HHV-2) (also referred to as herpes simplex virus type 2 (HSV-2)) of the Simplexvirus genus, Human herpesvirus 3 (HHV-3) (also referred to as varicella-zoster virus (VZV)) of the Varicellovirus genus, Human herpesvirus 4 (HHV-4) (also referred to as Epstein-Barr virus (EBV)) of the Lymphocryptovirus genus, Human herpesvirus 5 (HHV-5) (also referred to as cytomegalovirus (CMV)) of the Cytomegalovirus genus, Human herpesvirus 6 (HHV-6) and Human herpesvirus 7 (HHV-7) of the Roseolovirus genus, and Human herpesvirus 8 (HHV-8) (also referred to as Kaposi's sarcoma-associated herpesvirus (KSHV)) of the Rhadinovirus genus. HHV-6 includes Human betaherpesvirus 6A (HHV-6A) and Human betaherpesvirus 6B (HHV-6B). For example, as of January 2023, characteristics of herpesviruses (and genes thereof) may be referenced from publicly available information shown in the table below.

**[Table A]**

| | | |
|---|---|---|
| HHV-1 | Human alphaherpesvirus 1 isolate KOS | https://www.ncbi.nlm.nih.gov/nuccore/KT899744.1 |
| HHV-2 | Human alphaherpesvirus 2 strain MS | https://www.ncbi.nlm.nih.gov/nuccore/MK855052.1 |
| HHV-3 | Human herpesvirus 3 strain Ellen | https://www.ncbi.nlm.nih.gov/nuccore/JQ972913.1 |
| HHV-4 | Human gammaherpesvirus 4 | https://www.ncbi.nlm.nih.gov/nuccore/NC_007605.1 |
| HHV-5 | Human herpesvirus 5 strain Towne | https://www.ncbi.nlm.nih.gov/nuccore/FJ616285.1 |
| HHV-6A | Human betaherpesvirus 6A isolate U1102 | https://www.ncbi.nlm.nih.gov/nuccore/NC_001664.4 |
| HHV-6B | Human herpesvirus 6B | https://www.ncbi.nlm.nih.gov/nuccore/NC_000898.1 |
| HHV-7 | Human herpesvirus 7 | https://www.ncbi.nlm.nih.gov/nuccore/NC_001716.2 |
| HHV-8 | Human herpesvirus 8 strain GK18 | https://www.ncbi.nlm.nih.gov/nuccore/NC_009333.1 |

In the present specification, the term "adeno-associated virus" (AAV) refers to a linear single-stranded DNA virus of the genus Dependoparvovirus in the family Parvoviridae, and the viral particle has a diameter of 20 to 26 nm. Proliferation of AAV usually requires adenoviral elements. At the quantity termini of the AAV genome, there are T-shaped hairpin structures called inverted terminal repeats (ITRs). This part of the ITR serves as the origin of replication and functions as a primer. In addition, the ITR is also required for packaging into viral particles and for integration into chromosomal DNA of a host cell. In the left half of the genome, a rep gene encoding non-structural proteins, that is, regulatory proteins involved in replication and transcription (Rep78, Rep76, Rep52, and Rep40) is present, and in the right half of the genome, a cap gene encoding three capsid structural proteins (VP1, VP2, and VP3) is present. The life cycle of AAV is classified into latent infection and lytic infection. The former occurs when AAV infects alone, and is characterized by being incorporated into the AAVS1 region on the long arm of chromosome 19 (19q13.3-qter) of the host cell. This incorporation is due to non-homologous recombination and involves Rep. It has been reported that Rep78/Rep76 binds to a base sequence (GAGC repeat sequence) commonly present in the AAVS1 region and the Rep-binding region of ITR. Accordingly, when wild-type AAV infects a target cell, it is considered that Rep binds to both the ITR of AAV and the AAVS1 region, and that site-specific integration of the AAV genome into chromosome 19 occurs through the involvement of Rep. When a helper virus such as an adenovirus simultaneously infects a cell, or when a helper virus further superinfects a cell in which AAV is latently infected, replication of AAV occurs, and a large amount of virus is released due to cell lysis (lytic infection).

AAV has been reported to have serotypes 1 to 12 based on its capsid. In addition, serotypes such as rh10, DJ, DJ/8, PHP.eB, PHP.S, AAV2-retro, AAV2-QuadYF, AAV2.7m8, AAV6.2, rh.74, AAV2.5, AAV-TT, and Anc80 have also been reported. The AAV-derived construct of the present disclosure may be produced based on an AAV of a serotype suitable for the target tissue. For example, the relationship of (serotype): (target tissue) may be selected as follows: (AAV1): (muscle, liver, respiratory tract, neuronal cells); (AAV2):(muscle, liver, neuronal cells); (AAV3): (muscle, liver, neuronal cells); (AAV4): (muscle, ependymal cells); (AAV5): (muscle, liver, neuronal cells, glial cells, respiratory tract); (AAV6): (muscle, liver, respiratory tract, neuronal cells); (AAV7): (muscle, liver); (AAV8): (muscle, liver); and (AAV9): (muscle, liver, respiratory tract). As the capsid of AAV, in addition to the wild-type, examples thereof include capsids with targeting mutations (such as AAV2i8, AAV2.5, AAV-TT, and AAV9.HR), capsids with random mutations (such as AAV-PHP.B), and capsids designed in silico (such as Anc80), and in one embodiment, the AAV-derived construct of the present disclosure may contain these modified capsids, and the AAV-derived construct plasmid of the present disclosure may be constructed to encode these modified capsids. In the present specification, when a nucleic acid sequence is described as being derived from a specific serotype, it is intended that the nucleic acid sequence may encode a wild-type capsid or may encode a modified capsid based on the wild-type capsid as described above.

In the present specification, the term "virus-derived" for a nucleic acid means that the nucleic acid has high homology or identity to a nucleic acid present in the virus, and does not require that the nucleic acid be obtained from the virus or prepared using a nucleic acid obtained from the virus. For example, when a nucleic acid or a translation product thereof has a sequence having high identity (for example, at least 75%, at least 80%, at least 85%, at least 90%, at least 91%, at least 92%, at least 93%, at least 94%, at least 95%, at least 96%, at least 97%, at least 98%, or at least 99% identity) to a nucleic acid present in a virus or a translation product thereof, the nucleic acid may be considered to be derived from a virus.

In the present specification, the term "helper gene" refers to any gene that increases AAV yield in a producer cell. For example, in a case where HEK293 cells (ATCC: CRL-1573) are introduced with pAAV-ZsGreen (TAKARA: 6231) and pRC1 (TAKARA: 6672), and cells with or without the introduction of a candidate gene are prepared, these cells are cultured in DMEM (Thermo Fisher: 11965) (2% FBS (Thermo Fisher: 10270) and 1% penicillin-streptomycin mixed solution (NACALAI TESQUE, INC.: 09367-34)) medium at 37°C in a 5% CO₂ incubator, after 3 days, 5 µL of Nuclease-Free Water (Promega: P1193), 1 µL of 10 × React Buffer (with MgCl₂) (Thermo Fisher: ED0521), and 2 µL of DNase I (Thermo Fisher: ED0521) are added to 2 µL of the collected supernatant and reacted at 37°C for 30 minutes in a block incubator, then, 90 µL of Nuclease-Free Water (0.001% Pluronic (registered trademark) F-68 (100X) (Thermo Fisher: 24040032) and 5 mM EDTA (TAKARA: T9191)) are added and reacted at 95°C for 10 minutes in a block incubator, to 2 µL of the solution prepared by 100-fold dilution of the obtained solution with Nuclease-Free Water (0.001% Pluronic (registered trademark) F-68 (100X)), 2 µL of Nuclease-Free Water, 5 µL of PowerUp (registered trademark) SYBR (registered trademark) Green Master Mix (Thermo Fisher: A25780), 0.5 µL of 10 µM CMV-F primer (CATCAATGGGCGTGGATAGC: SEQ ID NO: 1), and 0.5 µL of 10 µM CMV-R primer (GGAGTTGTTACGACATTTTGGAAA: SEQ ID NO: 2) are added, after 95°C for 10 minutes, qPCR is performed using QuantStudio 3 Real-Time PCR System Fast96-well (Thermo Fisher: QS3-96F) under the conditions of repetition of 40 cycles of 95°C for 15 seconds, 55°C for 5 seconds, and 72°C for 30 seconds, and the amount of AAV particles is measured, when the amount of AAV particles in cells into which a candidate gene is introduced is increased by at least 50% compared to cells into which the candidate gene is not introduced, the candidate gene can be determined to be a helper gene. Typically, the helper gene is selected from the adenoviral E1A, E1B, E2A, VA, or E4, a portion thereof, or a variant thereof. To date, 52 antigenic types (serotypes) of human adenovirus have been identified. The helper genes of the present disclosure may be derived from an adenovirus of any antigenic type. The helper gene of the present disclosure or a gene product thereof (such as a protein) may have a sequence (a base sequence or an amino acid sequence) having high identity (for example, at least 75%, at least 80%, at least 85%, at least 90%, at least 91%, at least 92%, at least 93%, at least 94%, at least 95%, at least 96%, at least 97%, at least 98%, or at least 99% identity) to a helper gene of an adenovirus of any serotype or a gene product thereof (such as a protein). A gene or gene product that differs from, but has high identity with, the wild type may also be referred to as a variant in the present specification.

In the present specification, the term "gene associated with replication of a gene" refers to any gene that promotes replication of the AAV genome. For example, pAAV-ZsGreen (TAKARA: 6231), pHelper (TAKARA: 6230), and a plasmid expressing Rep78 derived from AAV2 are introduced into HEK293 cells (ATCC: CRL-1573), cells with introduction of a candidate gene or cells without introduction of a candidate gene are prepared, these cells are cultured in DMEM (Thermo fisher: 11965) (2% FBS (Thermo fisher: 10270), 1% penicillin-streptomycin mixed solution (NACALAI TESQUE, INC.: 09367-34)) medium in an incubator at 37°C with 5% CO₂, after 2 days, the cells are detached with a cell scraper, the cells are collected by centrifugation, lysis buffer (2 mM MgCl₂ (NACALAI TESQUE, INC.: 09884-12), 150 mM NaCl (NACALAI TESQUE, INC.: 09649-15), 50 mM Tris (NACALAI TESQUE, INC.: 02435-15), and 0.1% Triton X-100 (NACALAI TESQUE, INC.: 12969-25); pH 8.5) is added to the resulting cell pellet to suspend it, and the suspension is reacted in a block incubator at 37°C for 30 minutes. In a case where 2 µL of Nuclease-Free water, 5 µL of PowerUp (registered trademark) SYBR (registered trademark) Green Master Mix (Thermo fisher: A25780), 0.5 µL of 10 µM CMV-F primer (CATCAATGGGCGTGGATAGC: SEQ ID NO: 1), and 0.5 µL of 10 µM CMV-R primer (GGAGTTGTTACGACATTTTGGAAA: SEQ ID NO: 2) are added to 2 µL of a solution prepared by 1,000-fold dilution of 2 µL of a supernatant collected by centrifugation, and after 95°C for 2 minutes, qPCR is performed using QuantStudio 3 Real-Time PCR System Fast96-well (Thermo Fisher: QS3-96F) under the conditions of repetition of 40 cycles of 95°C for 15 seconds, 55°C for 5 seconds, and 72°C for 30 seconds, when the amount of AAV genome is measured, if the amount of AAV genome in the cells into which the candidate gene is introduced is increased by at least 50% compared to the cells into which the candidate gene is not introduced, the candidate gene can be determined to be a gene associated with replication of the gene. Examples of genes associated with replication of the genes include, typically, UL8, UL5, UL52, UL29 (ICP8), UL30, and UL42 of HHV-1, UL5, UL8, UL9, UL29, UL30, UL42, and UL52 of HHV-2, ORF6, ORF16, ORF28, ORF29, and ORF55 of HHV-3, BMRF1, BSLF1, BBLF4, BBLF2/BBLF3, BALF5, and BALF2 of HHV-4, UL102, UL105, UL70, UL57, UL54, UL44, and UL112 of HHV-5, U27, U38, and U41 of HHV-6A, U38, U41, and U79 of HHV-6B, U27, U38, U41, U43, U73, U74, U77, and U79 of HHV-7, and ORF6, ORF9, and ORF59 of HHV-8. The gene associated with replication of the gene or the gene product (such as a protein) thereof according to the present disclosure may have a sequence (a base sequence or an amino acid sequence) having high identity (for example, at least 75%, at least 80%, at least 85%, at least 90%, at least 91%, at least 92%, at least 93%, at least 94%, at least 95%, at least 96%, at least 97%, at least 98%, or at least 99% identity) to a gene associated with replication of a gene of any wild-type herpesvirus or a gene product thereof.

In the present specification, when a sequence is within a defined identity range (for example, at least 90%) with a plurality of genes or gene products thereof, the sequence may be associated with the gene or gene product thereof that exhibits the highest identity. For example, when a sequence is 98% identical to the base sequence of gene A and 95% identical to the base sequence of gene B, the sequence may be referred to as a variant of gene A.

In the present specification, the phrase "two genes are in cis" refers to a state in which these genes are present on the same nucleic acid molecule or a nucleic acid molecule of a complementary strand (in the case of double-stranded nucleic acid). In addition, in the present specification, the phrase "two genes are in trans" refers to a state in which, in a certain cell (in a certain organism), these genes are not present on the same nucleic acid molecule or a nucleic acid molecule of a complementary strand (in the case of double-stranded nucleic acid). For example, a gene present on the genome and a gene on a nucleic acid introduced by a virus-derived construct may be in trans. If necessary, whether two genes are in trans may be determined based on the state at the time when the two genes come to be simultaneously present in a cell (for example, by introduction of a nucleic acid into the cell).

In the present specification, when referring to the number of genes, a single gene refers to a gene that has a contiguous sequence in a form that is normally (with the highest frequency or a probability of 50% or more) present on the genome of a certain organism. For example, two exons encoding a certain protein may be two genes. For example, when a promoter sequence and a sequence encoding a protein form a continuous sequence, a nucleic acid segment including the promoter sequence and the sequence encoding the protein may be a single gene. For example, when a protein that becomes functional upon cleavage is encoded by a continuous sequence on the genome, the protein may be encoded by a single gene. When referring to a gene in terms of function, the nucleic acid sequence is not required to be a continuous sequence, and, for example, a plurality of exons encoding a certain protein are collectively referred to as the gene for the protein.

As used in the present specification, the "deficient" of a gene refers to that a nucleic acid does not contain the gene or contains a gene that is modified so as not to perform the normal function (for example, the function of producing a functional protein) of the gene.

As used in the present specification, the "operably linked" refers to placing the expression (actuation) of a desired sequence under the control of a transcriptional and translational regulatory sequence (for example, a promoter, an enhancer, or the like) or a translational regulatory sequence. In order for a promoter to be operably linked to a gene, the promoter is usually located immediately upstream of the gene, but is not necessarily located adjacent to the gene.

As used in the present specification, the "plasmid" refers to a circular DNA that is present separately from chromosomes in a cell or that is present separately from chromosomes when introduced into a cell.

As used in the present specification, the term "cationic substance" refers to a substance that carries a positive charge when added to neutral water, and is typically provided in a complex with a nucleic acid (such as a plasmid).

In the present specification, the term "virus-derived construct" refers to a construct that includes a nucleic acid construct having a structure at least partially derived from a virus or a protein produced therefrom. Examples of the virus-derived construct include viral vectors, virus-like particles (VLPs), oncolytic viruses (including those modified to proliferate only in cancer cells), and viral replicons (self-replicating viral genomes lacking the ability to produce viral particles).

As used in the present specification, the term "viral vector" refers to a construct that at least partially has a structure derived from a virus and is capable of introducing a nucleic acid into a target cell. Typically, a viral vector is in the form of a viral particle containing a viral structural protein and a nucleic acid containing a heterologous gene.

As used in the present specification, the term "producer cell" refers to a cell capable of producing a desired virus-derived construct, and may be a cell in which genes necessary for production of the virus-derived construct are expressed from a chromosome and an introduced plasmid. A desired virus-derived construct may be produced by introducing a plasmid into a producer cell. For example, in the case of an AAV viral vector, E1A and E1B are required for its production, but since HEK293 cells have these, they can be omitted from the helper plasmid. Other cells also function as producer cells when modified to have such functional auxiliary factors.

In the present specification, the term "animal cell" refers to a cell obtained from an animal (such as a human, mouse, rat, monkey, or dog), or a cell modified from a cell obtained from an animal.

As used in the present specification, the "host cell" refers to a cell into which an exogenous nucleic acid, protein, virus, or virus-derived construct is introduced (including the progeny of such a cell).

In the present specification, the "protein", "polypeptide", and "peptide" are used in the same meaning, and refer to an amino acid polymer of any length. The polymer may be linear, branched, or cyclic. An amino acid may be a natural, non-natural, or modified amino acid. The term also encompasses a natural or artificially modified polymer. Examples of such a modification include disulfide bond formation, glycosylation, lipidation, acetylation, phosphorylation, and any other manipulation or modification (for example, conjugation with a labeling component).

As used in the present specification, the term "transcriptional and translational regulatory sequence" collectively refers to promoter sequences, polyadenylation signals, transcription termination sequences, upstream regulatory domains, origins of replication, enhancers, IRES, and the like, which cooperate to enable replication, transcription, and translation of a coding sequence in a recipient cell. Not all of the transcriptional and translational regulatory sequences are necessarily required, as long as replication, transcription, and translation of the selected coding sequence are possible in a suitable host cell. Those skilled in the art can readily identify a regulatory nucleic acid sequence from publicly available information. Furthermore, those skilled in the art can identify a transcriptional and translational regulatory sequence applicable to the intended use, for example, in vivo, ex vivo, or in vitro.

As used in the present specification, the term "promoter" refers to a segment of a nucleic acid sequence that controls transcription of an operably linked nucleic acid sequence. The promoter includes a specific sequence sufficient for recognition, binding, and initiation of transcription by RNA polymerase. The promoter may have a sequence that regulates recognition, binding, or initiation of transcription by RNA polymerase.

As used in the present specification, the term "enhancer" refers to a segment of a nucleic acid sequence that functions to enhance the expression efficiency of a target gene.

As used in the present specification, the term "silencer" refers to a segment of a nucleic acid sequence that functions, in contrast to an enhancer, to reduce the expression efficiency of a target gene.

As used in the present specification, the term "insulator" refers to a segment of a nucleic acid sequence that has a cis-regulatory function of regulating the expression of genes located at distant positions on the DNA sequence.

As used in the present specification, the term "terminator" refers to a segment of a nucleic acid sequence that is located downstream of a protein-coding region and is involved in the termination of transcription when the nucleic acid is transcribed into mRNA.

As used in the present specification, the "origin of replication" refers to a segment of a nucleic acid sequence to which a protein (for example, initiator DnaA protein or the like) that recognizes the nucleic acid sequence binds or to which RNA is synthesized to partially unwind a DNA double helix, and from which replication is initiated.

As used in the present specification, an internal ribosome entry site ("IRES") refers to a nucleic acid segment that promotes the entry or retention of a ribosome during translation of a downstream nucleic acid sequence.

In the present specification, the "homology" of nucleic acids refers to a degree of identity of two or more nucleic acid sequences with respect to one another, and having "homology" generally refers to having a high degree of identity or similarity. Therefore, the identity or similarity of the sequences is higher as homology of two nucleic acids is high. The "similarity" is a numerical value calculated for a similar base in addition to identity, and here, the similar bases refer to a case where some bases in a mixed base (for example, R = A + G, M = A + C, W = A + T, S = C + G, Y = C + T, K = G + T, H = A + T + C, B = G + T + C, D = G + A + T, V = A + C + G, and N = A + C + G + T) are identical. Whether two types of nucleic acids have homology may be determined by a direct comparison of sequences or a hybridization method under stringent conditions. When two nucleic acid sequences are directly compared, the genes have homology when the genes are typically at least 50% identical, preferably at least 70% identical, and more preferably, at least 80%, 90%, 95%, 96%, 97%, 98%, or 99% identical between the nucleic acid sequences.

Amino acids may be mentioned in the present specification by either their commonly known three letter symbols or their one character symbols recommended by the IUPAC-IUB Biochemical Nomenclature Commission. Similarly, nucleotides may be mentioned by their commonly recognized one character codes. In the present specification, a comparison of similarity, identity, and homology of an amino acid sequence and a base sequence is calculated by using a sequence analysis tool BLAST and default parameters. For example, identity can be searched using BLAST 2.10.1+ (published on June 18, 2020) of the NCBI. In the present specification, a value for identity generally refers to a value obtained when aligned under the default conditions using BLAST described above. However, when a higher value is obtained by changing a parameter, the highest value is set as the value of identity. In a case where identity is evaluated in a plurality of regions, the highest value in the plurality of regions is set as the value of identity. Similarity is a numerical value calculated by taking into consideration a similar amino acid in addition to identity.

In the present specification, unless otherwise specified, it is understood that reference to a biological material (for example, a protein, a nucleic acid, or a gene) is also a reference to a variant of the biological material (for example, a variant having a modification in a base sequence) that performs a function similar to (but not to the same extent as) the biological function of the biological material. Such a variant may contain a fragment of the original molecule, and a molecule that is at least 30%, 40%, 50%, 60%, 70%, 80%, 90%, 95%, 98%, or 99% identical as compared to the sequence of the original molecule that is aligned over the amino acid sequence or nucleic acid sequence of the original biological material of the same size, or aligned by computer homology programs known in the art. The variant may include molecules having modified nucleotides (for example, modification by methylation).

In the present specification, the "corresponding" amino acid or nucleic acid refers to an amino acid or a nucleotide which has or is expected to have, in a certain polypeptide molecule or polynucleotide molecule, a similar action as a predetermined amino acid or nucleotide in a reference polypeptide or a polynucleotide for comparison, and, particularly in the case of enzyme molecules, refers to an amino acid which is present at a similar position in an active site and makes a similar contribution to catalytic activity. For example, for an antisense molecule, it may be a similar moiety in an ortholog corresponding to a specific moiety of the antisense molecule. A corresponding amino acid may be a specific amino acid subjected to, for example, cysteination, glutathionylation, S-S bond formation, oxidation (for example, oxidation of a methionine side chain), formylation, acetylation, phosphorylation, glycosylation, myristylation, or the like. Alternatively, a corresponding amino acid may be an amino acid responsible for dimerization. Such a "corresponding" amino acid or nucleic acid may be a region or a domain over a certain range. Accordingly, it is referred in the present specification as a "corresponding" region or domain in such a case.

In the present specification, the "corresponding" gene (for example, a polynucleotide sequence or molecule) refers to a gene (for example, a polynucleotide sequence or molecule) in a certain species which has or is expected to have a similar action as a predetermined gene in a reference species for comparison. When a plurality of genes having such an action are present, the corresponding gene refers to a gene having the same evolutionary origin. Accordingly, a gene corresponding to a certain gene may be an ortholog of such a gene. For example, the cap of AAV serotype 1 may correspond to the cap of AAV serotype 2. For example, a corresponding gene in a certain virus can be found by search on a sequence database for the virus using the gene sequence of a reference virus of the corresponding gene as a query sequence.

In accordance with the present disclosure, in the present specification, the term "activity" refers to a function of a molecule in its broadest sense. Activity, although not intended to be limiting, generally includes a biological function, biochemical function, physical function, and chemical function of a molecule. Examples of the activity include enzymatic activity, an ability to interact with another molecule, an ability to activate, promote, stabilize, inhibit, suppress, or destabilize a function of another molecule, stability, and an ability to localize at a specific position in a cell. When applicable, the term also relates to a function of a protein complex in the broadest sense.

In the present specification, when referring to a certain gene or a nucleic acid molecule or a polypeptide related thereto, the "biological function" refers to a specific function that the gene, nucleic acid molecule, or polypeptide may have in vivo, and examples thereof include, but are not limited to, a specific cell surface structure recognition ability, an enzyme activity, and a binding ability to a specific protein. In the present disclosure, for example, a function in which a certain promoter is recognized in a specific host cell can be exemplified, but the present disclosure is not limited thereto. In the present specification, the biological function may be exerted by "biological activity". In the present specification, the "biological activity" refers to an activity that a certain agent (for example, a polynucleotide, a protein, or the like) may have in a living body. Biological activity encompasses an activity of exerting a variety of functions (for example, transcription promoting activity), and also encompasses, for example, an activity of activating or inactivating another molecule by an interaction with a certain molecule. For example, when a certain factor is an enzyme, the biological activity thereof encompasses enzyme activity thereof. In another example, in a case where a certain factor is a ligand, binding to a receptor corresponding to the ligand is encompassed. Such biological activity can be measured by a technique that is well known in the art.

As used in the present disclosure, the term "infectivity" of a virus or a virus-derived construct refers to the ability of the virus or the virus-derived construct to introduce nucleic acid contained therein into a cell through attachment to the cell or membrane fusion of the virus or the virus-derived construct. The term "replicative ability" of a virus or a virus-derived construct refers to the ability to produce infectious virus particles or virus-derived construct particles in an infected cell.

As used in the present specification, the terms "transformation", "transduction", and "transfection" are used interchangeably, unless otherwise stated, and refer to the introduction of a nucleic acid into a host cell (optionally via a virus or a virus-derived construct). As a transformation method, any method can be used as long as it is a method of introducing a nucleic acid into a host cell, and examples thereof include various well-known techniques such as use of competent cells, an electroporation method, a method using a particle gun (gene gun), and a calcium phosphate method.

In the present specification, the "purified" substance or biological factor (for example, a nucleic acid, a protein, or the like) refers to a substance or a biological factor in which at least a part of a factor naturally accompanying the biological factor is removed. Thus, the purity of the biological factor in the purified biological factor is generally higher than the purity in the normal state of the biological factor (that is, concentrated). The "purified" as used in the present specification refers to the presence of preferably at least 75% by weight, more preferably at least 85% by weight, still more preferably at least 95% by weight, and most preferably at least 98% by weight of a biological factor of the same type. The substance used in the present disclosure is preferably a "purified" substance.

In the present specification, the term "pharmaceutical ingredient" refers to any component that can constitute a pharmaceutical composition, and examples thereof include active ingredients (which themselves exhibit a pharmacological effect), and additive ingredients (which themselves are not expected to exhibit a pharmacological effect but are expected to play certain roles (for example, excipients, lubricants, surfactants, and the like) when contained as a medicine). The pharmaceutical ingredient may be a single substance or a combination of a plurality of substances and agents. Any combination, such as a combination of an active ingredient and an additive ingredient, or a combination of an adjuvant and an active ingredient, may also be included.

In the present specification, the term "active ingredient" refers to an ingredient that exerts an intended pharmacological effect, and may refer to a single component or a plurality of components.

In the present specification, the term "additive ingredient" refers to any component that is not expected to exert a pharmacological effect but plays a certain role when contained as a medicine, and examples thereof include pharmaceutically acceptable carriers, stabilizers, (co)adjuvants, solubility enhancers, solubilizers, diluents, excipients, buffers, binders, diluents, flavoring agents, and lubricants.

In the present specification, a "drug", "agent", or "factor" (all the terms correspond to an "agent" in English) is used interchangeably in a broad sense, and may be any substance or other elements (for example, energy such as light, radioactivity, heat, and electricity) as long as the intended object can be achieved. Examples of such a substance include, but are not limited to, a protein, a polypeptide, an oligopeptide, a peptide, a polynucleotide, an oligonucleotide, a nucleotide, a nucleic acid (including, for example, DNA such as cDNA or genomic DNA, RNA such as mRNA), a polysaccharide, an oligosaccharide, a lipid, an organic small molecule (for example, a hormone, a ligand, an information transmitting substance, an organic small molecule, a molecule synthesized by combinatorial chemistry, a small molecule that may be used as a medicine (for example, a small molecule ligand or the like), or the like), a composite molecule thereof, and a mixture thereof.

As used in the present specification, the "complex" or "complex molecule" means any construct containing two or more moieties. For example, when one moiety is a polypeptide, the other moiety may be a polypeptide or another substance (for example, a substrate, a sugar, a lipid, a nucleic acid, another hydrocarbon, or the like). In the present specification, two or more moieties constituting the complex may be bonded by a covalent bond or may be bonded by another bond (for example, a hydrogen bond, an ionic bond, a hydrophobic interaction, van der Waals force, or the like).

In the present specification, the term "label" refers to the presence (for example, a substance, energy, an electromagnetic wave, and the like) for identifying a molecule or substance to be targeted from others. Examples of such a labeling method include a radioisotope (RI) method, a fluorescence method, a biotin method, and a chemiluminescence method. When a plurality of target proteins or factors or means for capturing the same are labeled by a fluorescence method, the markers are labeled with fluorescent substances having different maximum fluorescence emission wavelengths. A difference in maximum fluorescence emission wavelength is preferably 10 nm or more. Any label that does not affect the function can be used, and examples of the fluorescent substance include Alexa^{™} Fluor. Alexa^{™} Fluor is a water-soluble fluorescent dye obtained by modifying coumarin, rhodamine, fluorescein, cyanine, or the like, is a series corresponding to a wide range of fluorescence wavelengths, and is significantly stable, bright, and less pH sensitive as compared with other fluorescent dyes having corresponding wavelengths. Examples of a combination of fluorescent dyes having a maximum fluorescence wavelength of 10 nm or more include a combination of Alexa^{™} 555 and Alexa^{™} 633 and a combination of Alexa^{™} 488 and Alexa^{™} 555. Examples of other fluorescent labels include cyanine dyes (for example, Cy3 and Cy5 of CyDye^{™} series, and the like), rhodamine 6G reagents, N-acetoxy-N2-acetylaminofluorene (AAF), and AAIF (an iodine derivative of AAF). In the present disclosure, such a label may be utilized to modify the object to be targeted so that the object may be detected by detection means used. Such modifications are known in the art and those skilled in the art can carry out such methods as appropriate for the label and depending on the object to be targeted.

In the present specification, the "kit" refers to a unit generally providing portions to be provided (for example, a virus-derived construct, instructions, and the like) that are usually divided into two or more sections. The form of the kit is preferred when it is intended to provide a composition that should not be provided in a mixed state for stability or the like, but is preferably mixed immediately before use. Such a kit preferably includes an instruction or manual describing how to use the provided portions or how a reagent should be processed. When the kit is used in the present specification as a reagent kit, the kit generally includes an instruction or the like describing how to use a plasmid and the like.

In the present specification, the "instruction" is a document with an explanation of the method of use of the present disclosure for a physician or other users. The instruction includes wording that instructs administration of the medicine or the like of the present disclosure. In addition, the instruction may include wording that instructs the administration form. The instruction is prepared in accordance with a format defined by the regulatory agency of the country in which the present disclosure is practiced (for example, the Ministry of Health, Labor and Welfare in Japan, Food and Drug Administration (FDA) in the United States or the like), with an explicit description showing approval by the regulatory agency. The instruction is a so-called package insert and is usually provided in, but is not limited to, a paper medium. For example, the instruction may also be provided in a form such as an electronic medium (for example, a web site or an e-mail provided on the Internet).

The term "about" refers to the indicated value plus or minus 10%. The "about" when used for a temperature refers to an indicated temperature plus or minus 5°C, and the "about" when used for a pH refers to an indicated pH plus or minus 0.5.

### (Preferred embodiments)

Preferred embodiments of the present disclosure will be described below. It is understood that the embodiments provided below are provided for a better understanding of the present disclosure, and that the scope of the present disclosure should not be limited to the following description. Therefore, it is apparent that those skilled in the art can appropriately make modifications within the scope of the present disclosure in view of the description in the present specification. In addition, it is also understood that the following embodiments may be used alone or in combination.

### (Virus-derived gene)

In one aspect, the present disclosure provides a virus-derived gene that can be used for producing a novel virus-derived construct. In one aspect, the virus-derived gene of the present disclosure includes a combination of an adenovirus-derived gene and a herpesvirus-derived gene. Typically, these genes are provided in a form contained in a nucleic acid molecule. A nucleic acid containing an adenovirus-derived gene and a nucleic acid containing a herpesvirus-derived gene (in the present specification, the nucleic acid is synonymous with a nucleic acid containing an adenovirus-derived gene and a herpesvirus-derived gene) may be the same nucleic acid molecule or different nucleic acid molecules, and when the nucleic acid forms a double strand, they may be contained in the same double-stranded nucleic acid complex or in different double-stranded nucleic acid complexes. In addition, in the present specification, any nucleic acid containing one or more elements (such as herpesvirus-derived genes) encompasses embodiments in which all of the elements are contained in the same nucleic acid molecule and embodiments in which all of the elements are contained in a combination of different nucleic acid molecules, and when the nucleic acid forms a double strand, the nucleic acid also encompasses embodiments in which all of the elements are contained in the same double-stranded nucleic acid complex and embodiments in which all of the elements are contained in a combination of different double-stranded nucleic acid complexes. Furthermore, in the present specification, any nucleic acid or gene may be provided as an isolated nucleic acid, may be provided as a part of a nucleic acid molecule, or may be provided in a state contained in a cell (for example, in a state integrated into a chromosome of the cell).

In one embodiment, the herpesvirus-derived gene is a gene associated with replication of a gene. In one embodiment, the herpesvirus-derived gene encodes a protein selected from a helicase, a primase, an ssDNA-binding protein (DBP), a DNA polymerase, and a DNA replication protein. In one embodiment, the herpesvirus-derived gene is selected from a DNA strand synthesis gene and a helicase-primase gene. Examples of the DNA strand synthesis gene include UL29 (ICP8), UL30, and UL42 of HHV-1, UL29, UL30, UL42, and UL52 of HHV-2, ORF16, ORF28, and ORF29 of HHV-3, BMRF1, BALF5, and BALF2 of HHV-4, UL57, UL54, and UL44 of HHV-5, U27, U38, and U41 of HHV-6A, U38, U41, and U79 of HHV-6B, U27, U38, and U41 of HHV-7, and ORF6, ORF9, and ORF59 of HHV-8. Examples of the helicase-primase gene include UL8, UL5, and UL52 of HHV-1, UL5, UL8, UL9, and UL52 of HHV-2, ORF6 and ORF55 of HHV-3, BSLF1, BBLF4, and BBLF2/BBLF3 of HHV-4, UL102, UL105, and UL70 of HHV-5, and U43, U73, U74, and U77 of HHV-7.

In one embodiment, the herpesvirus-derived gene includes a gene selected from UL8, UL5, UL52, UL29 (ICP8), UL30, UL42, UL102, UL105, UL70, UL57, UL54, UL44, and UL112. In one embodiment, the herpesvirus-derived gene includes a DNA strand synthesis gene, and in one embodiment, it is selected from UL29 (ICP8), UL30, UL42, UL57, UL54 and UL44. In one embodiment, the herpesvirus-derived gene includes only one of a DNA strand synthesis gene or a helicase-primase gene, in which each of the DNA strand synthesis gene or the helicase-primase gene may include one or more genes. In one embodiment, the herpesvirus-derived gene includes a gene derived from a herpesvirus selected from HHV-1 to HHV-8. In one embodiment, the herpesvirus-derived gene is selected from UL8, UL5, UL52, UL29 (ICP8), UL30, and UL42 of HHV-1, UL5, UL8, UL9, UL29, UL30, UL42, and UL52 of HHV-2, ORF6, ORF16, ORF28, ORF29, and ORF55 of HHV-3, BMRF1, BSLF1, BBLF4, BBLF2/BBLF3, BALF5, and BALF2 of HHV-4, UL102, UL105, UL70, UL57, UL54, UL44, and UL112 of HHV-5, U27, U38, and U41 of HHV-6A, U38, U41, and U79 of HHV-6B, U27, U38, U41, U43, U73, U74, U77, and U79 of HHV-7, and ORF6, ORF9, and ORF59 of HHV-8. In one embodiment, when the herpesvirus-derived gene includes a plurality of genes, the genes may be derived from different herpesviruses or the same herpesvirus among herpesviruses selected from HHV-1 to HHV-8.

In one embodiment, the adenovirus-derived gene includes a helper gene. In one embodiment, the adenovirus-derived gene includes a gene selected from E1A, E1B, E2, E4, and VA. In one embodiment, the adenovirus-derived gene is derived from an adenovirus of a serotype selected from serotypes 1 to 52.

In one embodiment, the adenovirus-derived gene and the herpesvirus-derived gene may be provided at a molar ratio of the adenovirus-derived gene to the herpesvirus-derived gene of about 100:1 to 1:5, for example, about 100:1 to 1:2, about 100:1 to 1:1, about 100:1 to 2:1, about 100:1 to 5:1, about 100:1 to 10:1, about 100:1 to 20:1, about 50:1 to 1:5, about 50:1 to 1:2, about 50:1 to 1:1, about 50:1 to 2:1, about 50:1 to 5:1, about 50:1 to 10:1, about 20:1 to 1:5, about 20:1 to 1:2, about 20:1 to 1:1, about 20:1 to 2:1, about 20:1 to 5:1, about 10:1 to 1:5, about 10:1 to 1:2, about 10:1 to 1:1, about 10:1 to 2:1, about 5:1 to 1:5, about 5:1 to 1:2, about 5:1 to 1:1, or about 5:1 to 2:1.

In one aspect, the virus-derived gene of the present disclosure is a gene associated with replication of a virus-derived gene. In one embodiment, the gene associated with replication of the virus-derived gene is provided in the form of a nucleic acid. In one embodiment, the virus-derived polymerase gene is derived from an adenovirus or a herpesvirus. In one embodiment, the virus-derived polymerase gene is derived from a herpesvirus. In one embodiment, the herpesvirus-derived polymerase gene is introduced into a cell (such as a producer cell) without being combined with an adenovirus-derived helper gene (for example, one or more or all selected from E1A, E1B, E2, E4, and VA) or with an adenovirus-derived polymerase gene. In one embodiment, the gene associated with replication of the herpesvirus-derived gene is selected from a DNA strand synthesis gene and a helicase-primase gene. In one embodiment, the gene associated with replication of the herpesvirus-derived gene includes a gene selected from UL8, UL5, UL52, UL29 (ICP8), UL30, UL42, UL102, UL105, UL70, UL57, UL54, UL44, and UL112. In one embodiment, the gene associated with replication of the herpesvirus-derived gene includes a DNA strand synthesis gene, and in one embodiment, it is selected from UL29 (ICP8), UL30, UL42, UL57, UL54 and UL44. In one embodiment, the gene associated with replication of the herpesvirus-derived gene includes only one of a DNA strand synthesis gene or a helicase-primase gene, in which each of the DNA strand synthesis gene or the helicase-primase gene may include one or more genes. In one embodiment, a gene associated with replication of a virus-derived gene is from a herpesvirus selected from HHV-1 to HHV-8. In one embodiment, the gene associated with replication of the virus-derived gene is derived from an adenovirus selected from serotypes 1 to 52. In one embodiment, the gene associated with replication of the herpesvirus-derived gene is selected from UL8, UL5, UL52, UL29 (ICP8), UL30, and UL42 of HHV-1, UL5, UL8, UL9, UL29, UL30, UL42, and UL52 of HHV-2, ORF6, ORF16, ORF28, ORF29, and ORF55 of HHV-3, BMRF1, BSLF1, BBLF4, BBLF2/BBLF3, BALF5, and BALF2 of HHV-4, UL102, UL105, UL70, UL57, UL54, UL44, and UL112 of HHV-5, U27, U38, and U41 of HHV-6A, U38, U41, and U79 of HHV-6B, U27, U38, U41, U43, U73, U74, U77, and U79 of HHV-7, and ORF6, ORF9, and ORF59 of HHV-8.

Any of the above virus-derived genes may include a nucleic acid having a sequence (base sequence or amino acid sequence) having at least 75%, at least 80%, at least 85%, at least 90%, at least 91%, at least 92%, at least 93%, at least 94%, at least 95%, at least 96%, at least 97%, at least 98%, or at least 99% identity to a wild-type gene or a translation product thereof, or a nucleic acid encoding the translation product.

The virus-derived genes of the present disclosure may be provided in combination with any additional elements for producing an adeno-associated virus-derived construct (such as a nucleic acid encoding Cap, a nucleic acid encoding Rep, or an ITR). The additional nucleic acid elements may be provided in the same nucleic acid molecule or the same double-stranded nucleic acid complex as the nucleic acid containing the virus-derived gene and/or the nucleic acid containing the helper gene of the present disclosure, or may be provided separately.

In one embodiment, the virus-derived gene of the present disclosure can be provided in combination with a nucleic acid containing a gene of interest between a 5' ITR and a 3' ITR. In one embodiment, the virus-derived gene of the present disclosure can be provided in combination with a nucleic acid containing a gene of interest, a promoter, and a terminator between a 5' ITR and a 3' ITR. In one embodiment, the virus-derived gene of the present disclosure includes AAV 5' ITR, rep, cap, assembly-activating protein (AAP), membrane-associated accessory protein (MAAP), and 3' ITR, as well as adenoviral E1A, E1B, E2A, VA, and E4. Any gene may be provided in a form that is present in a cell (such as a producer cell) or may be provided in a form that is integrated into the chromosome of the cell.

In one embodiment, the nucleic acid containing the virus-derived gene of the present disclosure may be provided in the form of a complex with a cationic substance. Examples of the cationic substance include PEI pro, FectoVIR-AAV, Lipofectamine 2000, Lipofectamine 3000, FuGENE 6, FuGENE HD, and FuGENE 4K.

In one embodiment, any nucleic acid of the present disclosure may include a promoter upstream of the gene, as necessary. In one embodiment, any nucleic acid of the present disclosure can contain a drug selection marker nucleic acid sequence.

A virus (for example, AAV)-derived construct produced using the virus-derived gene of the present disclosure contains structural proteins (such as a capsid), which may be involved in binding to a tissue or cell. Therefore, the targeting of the virus-derived construct to the tissue or cell can be adjusted by modifying the structural protein to change its binding affinity to a tissue or cell (or a surface structure thereof). In one embodiment, the virus-derived gene of the present disclosure may be provided in combination with a nucleic acid containing a gene encoding a structural protein, and the structural protein may be modified. Examples of a modification of a structural protein to adjust tissue or cell targeting include substitution or fusion with another protein (such as capsids of other viruses (for example, capsids of viruses of other serotypes, VSV-G), antigen-binding regions of antibodies, or ligand proteins of the subject organism (ligands for cancer antigens)). In addition, a virus-derived construct having an envelope may also contain cell membrane components of the producer cell in the envelope. Therefore, the targeting of the virus-derived construct having an envelope to a tissue or cell can be adjusted by modifying the cell membrane components of the producer cell. In one embodiment, the virus-derived construct may be designed to target nerve cells (cells of the peripheral nervous system or central nervous system, brain cells such as neurons and oligodendrocytes, and the like), lung cells, ocular cells (retinal cells, retinal pigment epithelium, corneal cells, and the like), epithelial cells (for example, epithelial cells of the intestine or respiratory tract), muscle cells (for example, skeletal muscle cells, cardiomyocytes, smooth muscle cells, and diaphragm muscle cells), dendritic cells, pancreatic cells (for example, islet cells), hepatocytes, cardiomyocytes, bone cells (for example, bone marrow stem cells), hematopoietic stem cells, spleen cells, keratinocytes, fibroblasts, endothelial cells, prostate cells, germ cells, cancer cells, and the like. A surface structure (such as a receptor) specific to each cell is known, and those skilled in the art can appropriately select a protein that strongly or specifically binds to the surface structure.

In one embodiment, the virus-derived gene of the present disclosure may be provided in combination with a nucleic acid containing a gene encoding an attenuated protein of a protein derived from the origin virus of the virus-derived construct to be produced. A gene encoding any known attenuated viral proteins may be included.

In one embodiment, the virus-derived gene of the present disclosure may be provided in combination with a nucleic acid containing a gene of interest. In one embodiment, the gene of interest can be positioned between two terminal repeats. The gene of interest may ultimately be incorporated into a virus-derived construct. Such a gene of interest may encode a therapeutic protein, may encode a gene for gene therapy, may encode a gene for gene and cell therapy such as CAR-T therapy, may encode a non-protein-coding functional RNA (such as rRNA, tRNA, microRNA (miRNA), or lncRNA), and may include transcriptional and translational regulatory sequences such as a promoter, a terminator, an insulator, an enhancer, a silencer, and a replication origin in combination or independently thereof. In one embodiment, the gene of interest includes a viral promoter such as a cytomegalovirus promoter, a CAG promoter, an SV40 promoter, or an RSV promoter (if necessary, upstream of the protein-coding gene). In one embodiment, the gene of interest includes an IRES (if necessary, upstream of the protein-coding gene). In one embodiment, the gene of interest can be integrated into a chromosome of a subject to whom the virus-derived construct is administered. In the embodiment, the gene of interest may have a function of regulating the expression of a gene originally possessed by the subject, or may result in long-term expression of a protein. For example, a virus-derived construct based on an adeno-associated virus can be incorporated into the chromosome of the subject. In one embodiment, the gene of interest may be integrated into a therapeutic cell (for example, a chromosome) (for example, via ex vivo treatment of the cell with a virus-derived construct).

In one embodiment, the gene of interest may include a plurality of genes. In one embodiment, the gene of interest can include 2 to 100, for example, 2 or more, 3 or more, 4 or more, 5 or more, 7 or more, 10 or more, 12 or more, 15 or more, 20 or more, 25 or more, 30 or more, 40 or more, or 50 or more genes, and 100 or less, 90 or less, 80 or less, 70 or less, 60 or less, 50 or less, 40 or less, 35 or less, 30 or more, 25 or less, 20 or less, 17 or less, 15 or less, 12 or less, or 10 or less genes. In one embodiment, the gene of interest may have a base length of about 0.1 to 1,000 kbp, for example, about 0.1 kbp or more, about 0.3 kbp or more, about 1 kbp or more, about 2 kbp or more, about 5 kbp or more, about 7 kbp or more, about 10 kbp or more, about 20 kbp or more, about 50 kbp or more, or about 100 kbp or more, and about 1,000 kbp or less, about 700 kbp or less, about 500 kbp or less, about 200 kbp or less, about 100 kbp or less, about 70 kbp or less, about 50 kbp or less, about 20 kbp or less, or about 10 kbp or less. In one embodiment, the gene of interest includes a plurality of genes, thereby enabling the delivery of a nucleic acid with high functionality to the subject, such as a combination of a tissue-specific (for example, cancer tissue-specific) or time-specific promoter of the subject and a therapeutic gene (such as a therapeutic protein coding sequence, a gene for gene therapy, or a gene for gene cell therapy) operably linked thereto, or a sequence encoding a series of enzymes that enables coordinated expression of a series of enzymes that regulate the metabolic cascade.

In one embodiment, examples of the protein encoded by the gene of interest include a therapeutic polypeptide (for example, replacement therapy), an immunogenic polypeptide (for example, a pathogen polypeptide, a cancer antigen polypeptide), and the like. Examples of the therapeutic polypeptide include cystic fibrosis transmembrane conductance regulator (CFTR), dystrophin (mini-dystrophin and micro-dystrophin), myostatin propeptide, follistatin, activin type II soluble receptor, IGF-1, anti-inflammatory polypeptides, sarcospan, utrophin, mini-utrophin, coagulation factors (for example, factor VIII, factor IX, factor X, and the like), erythropoietin, angiostatin, endostatin, catalase, tyrosine hydroxylase, superoxide dismutase, leptin, LDL receptor, lipoprotein lipase, ornithine transcarbamylase, β-globin, α-globin, spectrin, α1-antitrypsin, adenosine deaminase, hypoxanthine guanine phosphoribosyltransferase, β-glucocerebrosidase, sphingomyelinase, lysosomal hexosaminidase A, branched-chain ketoacid dehydrogenase, RP65 protein, cytokines (for example, α-interferon, β-interferon, interferon-v, interleukin-2, interleukin-4, granulocyte-macrophage colony-stimulating factor, lymphotoxin, and the like), peptide growth factors, neurotrophic factors and hormones (for example, somatotropin, insulin, insulin-like growth factors 1 and 2, platelet-derived growth factor, epidermal growth factor, fibroblast growth factor, nerve growth factor, neurotrophic factors-3 and -4, brain-derived neurotrophic factor, bone morphogenetic protein, glial-derived growth factor, transforming growth factors-a and -β, and the like), lysosomal acid α-glucosidase, α-galactosidase A, tumor necrosis factor-α soluble receptor, S100A1, parvalbumin, adenylate cyclase type 6, anti-inflammatory factors, anti-myostatin protein, aspartoacylase, suicide gene products (for example, thymidine kinase, cytosine deaminase, diphtheria toxin, and tumor necrosis factor), tumor suppressor gene products (for example, p53, Rb, and Wt-1), TRAIL, and FAS-ligand.

Examples of the pathogen polypeptide include cell surface proteins of pathogenic organisms such as bacteria, fungi, and parasites, and proteins expressed on the surface of viruses (for example, spike proteins, envelope proteins, capsid proteins, and the like). Specific examples of the pathogen polypeptide include orthomyxovirus immunogens (for example, influenza virus hemagglutinin (HA), nucleoprotein), lentivirus immunogens (for example, HIV or SIV envelope GP160 protein, matrix/capsid proteins, gag, pol, env gene products), arenavirus immunogens (for example, Lassa virus nucleocapsid protein, envelope glycoproteins), poxvirus immunogens (for example, vaccinia L1 or L8 gene products), flavivirus immunogens (for example, yellow fever virus or Japanese encephalitis virus immunogens), filovirus immunogens (for example, Ebola virus or Marburg virus immunogens, such as NP and GP gene products), bunyavirus immunogens (for example, RVFV, CCHF, SFS virus immunogens), coronavirus immunogens (for example, human coronavirus immunogens such as human coronavirus envelope glycoprotein), polio immunogens, herpesvirus immunogens (for example, CMV, EBV, HSV immunogens), mumps virus immunogens, measles virus immunogens, rubella virus immunogens, diphtheria toxin or other diphtheria immunogens, pertussis antigens, and hepatitis (for example, hepatitis A, B, C, and the like) immunogens.

Examples of the cancer antigen polypeptide include BRCA1 gene products, BRCA2 gene products, gp100, tyrosinase, GAGE-1/2, BAGE, RAGE, LAGE, NY-ESO-1, CDK-4, β-catenin, MUM-1, caspase-8, KIAA0205, HPVE, SART-1, PRAME, p15, melanoma tumor antigens, MART-1, gp100 MAGE-1, MAGE-2, MAGE-3, CEA, TRP-1, TRP-2, P-15, tyrosinase, HER-2/neu gene products, CA125, LK26, FB5 (endosialin), TAG72, AFP, CA19-9, NSE, DU-PAN-2, CA50, SPan-1, CA72-4, HCG, STN (sialyl Tn antigen), c-erbB-2 protein, PSA, L-CanAg, estrogen receptor, milk fat globulin, p53 tumor suppressor protein, mucin antigens, telomerase, nuclear matrix proteins, prostate acid phosphatase, and papillomavirus antigens.

In one embodiment, the gene of interest may be a gene for treating a specific disease (for example, a gene therapy gene). The gene to be selected for a specific disease may be appropriately selected by those skilled in the art. Examples of such diseases include infectious diseases caused by various pathogens, cystic fibrosis, hemophilia A, hemophilia B, thalassemia, anemia, Alzheimer's disease, multiple sclerosis, Parkinson's disease, Huntington's disease, amyotrophic lateral sclerosis, spinal muscular atrophy, epilepsy, cancer (melanoma, adenocarcinoma, thymoma, lymphoma, sarcoma, lung cancer, liver cancer, colon cancer, leukemia, uterine cancer, breast cancer, prostate cancer, ovarian cancer, cervical cancer, bladder cancer, kidney cancer, pancreatic cancer, brain cancer, or the like), diabetes, muscular dystrophy, Gaucher disease, Hurler disease, adenosine deaminase deficiency, glycogen storage diseases, congenital emphysema, Lesch-Nyhan syndrome, Niemann-Pick disease, Tay-Sachs disease, Angelman syndrome, maple syrup urine disease, age-related macular degeneration, glaucoma, diabetic retinopathy, retinal degenerative diseases, astrocytoma, glioblastoma, heart failure, peripheral artery disease, arthritis, joint disorders, intimal hyperplasia, AIDS, muscle wasting, kidney deficiency, hepatitis, LDL receptor deficiency, hyperammonemia, Krabbe disease, Batten disease, spinocerebellar ataxia, phenylketonuria, autoimmune diseases, amino acid metabolism disorders, organic acid metabolism disorders, fatty acid metabolism disorders, mitochondrial diseases, carbohydrate metabolism disorders, lysosomal storage diseases, peroxisomal diseases, metal metabolism disorders, purine and pyrimidine metabolism disorders, vitamin metabolism disorders, neurotransmitter disorders, lipid metabolism disorders, connective tissue disorders, congenital porphyria, α1-antitrypsin deficiency, lysosomal storage diseases, mucopolysaccharidosis disorders, Fabry disease, Canavan disease, Leigh disease, Refsum disease, Tourette syndrome, primary lateral sclerosis, progressive muscular atrophy, Pick's disease, muscular dystrophy, myasthenia gravis, Binswanger's disease, cerebral infarction, mood disorders, depression, bipolar affective disorder, persistent mood disorder, secondary mood disorder, schizophrenia, substance dependence, anxiety disorders, obsessive-compulsive disorder, somatoform disorder, dissociative disorder, grief, postpartum depression, hallucinations, delusions, dementia, paranoia, autism spectrum disorder, attention deficit disorder, psychosexual disorders, sleep disorders, pain disorders, eating disorders, weight disorders, obesity, cachexia, anorexia nervosa, and bulimia. In one embodiment, the gene of interest may be integrated into a therapeutic cell (for example, a chromosome) used in therapies such as CART therapy (for example, via ex vivo treatment of the cell with a virus-derived construct).

### (Methods)

In one aspect, the present disclosure provides a method for producing a virus-derived construct, the method including: a step of introducing the nucleic acid containing the virus-derived gene of the present disclosure into a producer cell; and a step of forming a virus-derived construct in the producer cell. In one embodiment, at least a portion of the introduced nucleic acid is integrated into the chromosome of the producer cell.

### (Cells)

In one aspect, the present disclosure provides a cell containing the virus-derived gene of the present disclosure. Typically, the cell is a producer cell (for example, producing an AAV-derived construct). In one embodiment, the present disclosure provides a method for producing a cell, the method including a step of introducing at least one element of the virus-derived gene of the present disclosure into a cell.

Examples of the producer cell include, but are not limited to, human embryonic kidney cells (produced by transfecting human fetal kidney cells with DNA cleaved from adenovirus type 5), 911 cells, PER.C6 cells, E1-transformed amniotic cells, E1-transformed A549 cells, GH329: HeLa cells, HEK293 cells, IT293SF cells, HEK293T, HEK293F, Vero cells, CHO cells, Sf9 cells, FreeStyle (trademark) 293-F, Expi293-F (trademark), Expi293 inducible, Expi293 NGT-Viral Production Cells 1.0, Viral Production Cells 2.0 (VPC2.0 cells), AAVpro (registered trademark) 293T Cell Line, Lenti-X (trademark) 293T Cell Line, FreeStyle (trademark) CHO-S cells, ExpiCHO-S (trademark), VirusExpress (trademark) 293 AAV Production Cells, and VirusExpress (trademark) 293T Lentiviral Production Cells. Examples of the producer cell for producing an AAV-derived construct include, in particular, cells such as HEK293, HEK293T, HEK293F, Hela, and Sf9.

In one embodiment, the cell contains one or more of the virus-derived genes of the present disclosure in a chromosome. In one embodiment, the cell contains an inverted terminal repeat (ITR), a gene of interest (GOI), a Rep gene, and a Cap gene (optionally in a chromosome). In one embodiment, the cell contains a helper gene, an ITR, a GOI, a Cap gene, and a Rep gene (optionally in a chromosome). In one embodiment, the cell contains a helper gene, an ITR, a GOI, a Cap gene, and a Rep gene at a single locus in a chromosome.

### (Virus-derived construct)

In one aspect, the present disclosure also provides a virus-derived construct produced using the virus-derived gene of the present disclosure. Typically, the virus-derived construct is an AAV-derived construct. The virus-derived construct may be a viral vector, a virus-like particle (VLP), an oncolytic virus (including those modified to proliferate only in cancer cells), or a viral replicon (a self-replicating viral genome lacking the ability to produce viral particles).

The AAV-derived construct of the present disclosure may be produced based on an AAV of a serotype suitable for the target tissue. For example, the relationship of (serotype) : (target tissue) may be selected as follows: (AAV1) : (muscle, liver, respiratory tract, neuronal cells); (AAV2) : (muscle, liver, neuronal cells); (AAV3):(muscle, liver, neuronal cells); (AAV4):(muscle, ependymal cells); (AAV5):(muscle, liver, neuronal cells, glial cells, respiratory tract); (AAV6):(muscle, liver, respiratory tract, neuronal cells); (AAV7):(muscle, liver); (AAV8) : (muscle, liver); and (AAV9):(muscle, liver, respiratory tract). As the capsid of AAV, in addition to the wild-type, examples thereof include capsids with targeting mutations (such as AAV2i8, AAV2.5, AAV-TT, and AAV9.HR), capsids with random mutations (such as AAV-PHP.B), and capsids designed in silico (such as Anc80), and in one embodiment, the AAV-derived construct of the present disclosure may contain these modified capsids.

In one embodiment, the present disclosure provides a virus-derived construct-containing composition containing a population of the virus-derived constructs of the present disclosure. The virus-derived construct of the present disclosure produced by the method of the present disclosure, and the virus-derived construct-containing composition of the present disclosure may have structural features that are not fully analyzable or extremely difficult to analyze, such as the three-dimensional structure or surface modifications of the virus-derived constructs. Therefore, the feature of being produced by the method of the present disclosure is one of the features that appropriately represent the virus-derived construct of the present disclosure and the virus-derived construct-containing composition of the present disclosure.

In one embodiment, the present disclosure provides a virus-derived construct containing a nucleic acid containing the virus-derived gene of the present disclosure. The nucleic acid containing the virus-derived gene of the present disclosure is typically removed during the process of forming the virus-derived construct; however, complete removal may be difficult, and it may remain in the resulting virus-derived construct. When the virus-derived construct preparation is treated with DNase to degrade nucleic acids outside the viral particles, the DNase is inactivated, and nucleic acids are subsequently extracted from the viral particles, less than about 10 ng per dose of nucleic acid containing the virus-derived gene of the present disclosure (or fragments thereof, for example, fragments of 200 bp or less) may be detected.

### (Composition)

In one embodiment, the present disclosure provides a composition containing at least one of the virus-derived genes, nucleic acids, elements of expression control systems, cells, and virus-derived constructs described in the present specification. The composition may be a composition for producing a virus (for example, AAV)-derived construct.

### (Dosage form and the like)

The virus-derived genes, nucleic acids, cells, and virus-derived constructs described in the present specification may be provided in various forms (for example, compositions). The form of the composition may be, for example, an injection, a capsule, a tablet, a granule, an inhalant, or the like. An aqueous solution for injection may be stored, for example, in a vial or in a stainless container. In addition, the aqueous solution for injection may contain, for example, physiological saline, sugar (for example, trehalose), NaCl, NaOH, or the like.

In one embodiment, the composition of the present disclosure contains a pharmaceutically acceptable carrier or excipient. Such a carrier can be an aseptic liquid, for example, water or oil, includes a carrier derived from petroleum, an animal, a plant, or a synthetic origin, and includes, but is not limited to, peanut oil, soybean oil, mineral oil, sesame oil, and the like. When a medicine is orally administered, water is a preferred carrier. When the pharmaceutical composition is administered intravenously, saline and aqueous dextrose are preferred carriers. Preferably, an aqueous saline solution, and aqueous dextrose and a glycerol solution are used as a liquid carrier for an injectable solution. Examples of a suitable excipient include light anhydrous silicic acid, crystalline cellulose, mannitol, starch, glucose, lactose, sucrose, gelatin, malt, rice, wheat flour, chalk, silica gel, sodium stearate, glycerol monostearate, talc, sodium chloride, powdered skim milk, glycerol, propylene, glycol, water, ethanol, carmellose calcium, carmellose sodium, hydroxypropyl cellulose, hydroxypropyl methylcellulose, polyvinyl acetal diethylamino acetate, polyvinylpyrrolidone, gelatin, medium-chain fatty acid triglyceride, polyoxyethylene hydrogenated castor oil 60, poloxamer, saccharose, carboxymethylcellulose, corn starch, and an inorganic salt. The composition can contain a small amount of a wetting agent or an emulsifier, or a pH buffer, if desired. These compositions can be in the form of a solution, suspension, emulsion, tablet, pill, capsule, powder, sustained release mixture, or the like. The composition can also be formulated as a suppository using a traditional binding agent and carrier, for example, triglyceride. Oral formulation can also contain a standard carrier such as medicine grade mannitol, lactose, starch, magnesium stearate, sodium saccharin, cellulose, or magnesium carbonate. Examples of a preferred carrier are described in E. W. Martin, Remington's Pharmaceutical Sciences (Mark Publishing Company, Easton, U.S.A). In addition, for example, a surfactant, an excipient, a coloring agent, a flavoring agent, a preservative, a stabilizer, a buffer, a suspension, an isotonizing agent, a binder, a disintegrant, a lubricant, a fluidity accelerator, a corrigent, or the like may be contained. In one embodiment, the pH of any liquid composition of the present disclosure may be about 3, about 3.5, about 4, about 4.5, about 5, about 5.5, about 6, about 6.5, about 7, about 7.5, about 8, about 8.5, about 9, about 9.5, about 10, about 10.5, about 11, or within a range between any two of these values.

Any component of the composition of the present disclosure can be provided as a pharmaceutically acceptable salt, and examples thereof include salts formed with a free carboxyl group derived from hydrochloric acid, phosphoric acid, acetic acid, oxalic acid, tartaric acid, and the like, salts formed with a free amine group derived from isopropylamine, triethylamine, 2-ethylaminoethanol, histidine, procaine, and the like, and salts derived from sodium, potassium, ammonium, calcium, ferric hydroxide, and the like.

In a preferred embodiment, a composition can be formulated as a pharmaceutical composition adapted for administration to humans according to a known method. Such a composition can be administered by injection. A composition for use in injection administration is typically a solution in an aseptic isotonic aqueous buffer. The composition can also contain a solubilizing agent and a local anesthetic agent such as lidocaine which alleviates the pain at the site of injection as necessary. In general, the ingredients can be supplied separately or mixed and supplied together in a unit dosage form, and supplied as a lyophilized powder or water free concentrate, for example, in a sealed container such as an ampoule or sachet showing the amount of active agent. When the composition is to be administered by injection, the composition can be distributed by using an injection bottle containing aseptic agent-grade water or saline. When the composition is to be administered by injection, aseptic water or saline ampoule for injection can also be provided so that the ingredients can be mixed prior to administration.

### (Use and application)

The virus-derived genes, nucleic acids, cells, or virus-derived constructs described in the present specification, or compositions containing any of them, can be used in various applications, such as gene therapy, functional genomics, cancer vaccination, and/or antiviral vaccination.

When the virus-derived gene, nucleic acid, cell, or virus-derived construct of the present disclosure, or a composition containing any of them, is applied to a subject, the subject is not particularly limited, and may be a mammal (for example, mouse, rat, hamster, rabbit, cat, dog, cattle, sheep, pig, monkey, human), bird, reptile, amphibian, arthropod, fish, or the like.

The amount of the virus-derived gene, nucleic acid, cell, or virus-derived construct of the present disclosure, or a composition containing any of them, may vary depending on the nature of the disorder or condition to be treated or prevented, but can be determined by those skilled in the art based on the present specification using standard clinical techniques. An in vitro assay can be used in some cases to assist the identification of the optimal dosing range. The precise dose to be used in a formulation can also vary depending on the administration route or the severity of the disease or disorder. Thus, the dose should be determined in accordance with the judgment of the attending physician or the condition of each patient. The dosage of the virus-derived construct or the virus-derived construct-containing composition of the present disclosure is not particularly limited and may be, for example, 1 × 10⁵, 1 × 10⁶, 1 × 10⁷, 1 × 10⁸, 1 × 10⁹, 1 × 10¹⁰, 1 × 10¹¹, 1 × 10¹², 1 × 10¹³, 1 × 10¹⁴, or 1 × 10¹⁵ virus-derived constructs per administration, or within a range between any two of these values. The dosing interval is not particularly limited, and may be, for example, 1 or 2 administrations every 1, 7, 14, 21, or 28 days or 1 or 2 administrations per range of any two of these values. The dosage, dosing interval, and dosing method may be appropriately selected depending on the age, weight, symptom, target organ, or the like of the patient.

The administration route of the virus-derived gene, nucleic acid, cell, or virus-derived construct described in the present specification, or a composition containing any of them, may be, for example, intravenous, intradermal, subcutaneous, intramuscular, intraperitoneal, intrathecal, intraventricular, intracerebral, intrapulmonary, intranasal, epidural, or oral administration. In one embodiment, the compositions of the present disclosure can be used in combination with various delivery systems. Such a system includes, for example, encapsulation in liposomes, microparticles, and microcapsules; and the use of endocytosis mediated by a receptor. Administration of the medicine may also be performed via an appropriate route, for example, by injection, by bolus injection, by absorption through epithelial or mucosal linings (for example, oral, rectal, intestinal mucosa, or the like), and, if necessary, using an aerosolized formulation with an inhaler or nebulizer, and may also be administered in combination with other agents. The administration can also be systemic or local.

The virus-derived gene, nucleic acid, cell, or virus-derived construct of the present disclosure, or a composition containing any of them, may be provided as a kit. In one embodiment, the present disclosure provides a kit for producing a virus-derived construct, the kit including a nucleic acid containing a virus-derived gene. In one embodiment, the present disclosure provides a drug pack or kit including one or more containers filled with one or more components that may be added to the composition of the present disclosure. In some cases, information indicating approval of manufacture, use, or sale for administration to humans by a government agency regulating the manufacture, use, or sale of medicines or biological products in a stipulated form can be appended to such a container.

The formulation procedure for the composition the present disclosure as a medicine or the like is known in the art, and is described, for example, in the Japanese Pharmacopoeia, the United States Pharmacopeia, pharmacopeia of other countries, or the like. Accordingly, those skilled in the art can determine the embodiment, such as the amount to be used, without undue experimentation from the descriptions in the present specification.

In the present specification, "or" is used when "at least one or more" of the items listed in the text can be employed. The same applies to "or". When explicitly described in the present specification as "within the range" of "two values", the range also includes the two values themselves.

Reference literatures such as scientific literatures, patents, and patent applications cited in the present specification are incorporated herein by reference to the same extent that the entirety of each document is specifically described.

The present disclosure has been described above with reference to preferred embodiments for easy understanding. Hereinafter, the present disclosure will be described based on examples, but the above description and the following examples are provided for the purpose of illustration only and not for the purpose of limiting the present disclosure. Accordingly, the scope of the present disclosure is not limited to the embodiments or examples that are specifically described in the present specification, but is limited only by the claims. Examples

For reagents, the specific products described in the examples were used. However, the reagent can be substituted with an equivalent product from another manufacturer (Sigma-Aldrich, Wako Pure Chemical, NACALAI TESQUE, INC., R&D Systems, USCN Life Science INC, or the like).

### (Construction of plasmid DNA)

Each plasmid used in the present example was obtained or prepared as follows.

### • #1 SYNp148_UL5.8.52

The pUC19 vector plasmid was treated with the restriction enzymes (AatII and PciI). The following four fragments (a CMV promoter fragment, an SV40 polyA fragment, a bGH polyA fragment, and a CAG promoter fragment) were inserted therein using In-Fusion. The CMV promoter fragment was prepared using the CMV promoter gene sequence (VectorBuilder) as a template and the following primers: 5'-cgaaaagtgccacctgcggccgcaagctttctaggcgttacataacttacggtaa-3' (SEQ ID NO: 3) and 5'-GAATTCCCTGCAGGGCTCTTCagctctgcttatatagacctc-3' (SEQ ID NO: 4). The SV40 polyA fragment was prepared using the SV40 polyA signal gene sequence (VectorBuilder) as a template and the following primers: 5'-CCCTGCAGGGAATTCtaagatacattgatgag-3' (SEQ ID NO: 5) and 5'-ggaggatccaacttgtttattgcagcttata-3' (SEQ ID NO: 6). The bGH polyA fragment was prepared using the bGH polyA signal gene sequence (VectorBuilder) as a template and the following primers: 5'-caagttggatcctccccagcatgcctgctattc-3' (SEQ ID NO: 7) and 5'-ttgGCTAGCtgtacactgtgccttctagttgcc-3' (SEQ ID NO: 8). The CAG promoter fragment was prepared using the CAG promoter gene sequence (VectorBuilder) as a template and the following primers: 5'-caagttggatcctccccagcatgcctgctattc-3' (SEQ ID NO: 9) and 5'-gctggccttttgctcgcggccgcagatctacgcgtCCTAGgctcgacattgattattgac-3' (SEQ ID NO: 10). The resulting plasmid was treated with the restriction enzyme (AvrII). This fragment was ligated with the following two fragments (an EFA1 promoter fragment and an hGH polyA fragment) using In-Fusion. The EFA1 promoter fragment was prepared using the EFA1 promoter gene sequence (VectorBuilder) as a template and the following primers: 5'-caatgtcgagcCTAGGggctccggtgcccgtcagtg-3' (SEQ ID NO: 11) and 5'-GAAGAGCggtaccGGCGCGCCtcacgacacctgaaatggaa-3' (SEQ ID NO: 12). The hGH polyA fragment was prepared using the hGH polyA signal gene sequence (VectorBuilder) as a template and the following primers: 5'-CCggtaccGCTCTTCgggtggcatccctgtgaccc-3' (SEQ ID NO: 13) and 5'-tctacgcgtgtcgacaaggacagggaagggagcag-3' (SEQ ID NO: 14). The resulting plasmid (pUC19_CMVp-CAGp-EF1Ap) was treated with the restriction enzymes (SbfI and EcoRI). This fragment was ligated with the following UL5 fragment using In-Fusion to construct pUC19_CMVp-UL5 (#1.1). The UL5 fragment was prepared using the UL5 gene sequence (ATCC VR-1493D) as a template and the following primers: 5'-agagctGAAGAGCCCTGCAGGATGGCGGCGGCCGGCGGGGAGCGCCAGCTAG-3' (SEQ ID NO: 15) and 5'-atgtatcttaGAATTCTTAATAGACAATGACCACGTTCGGATCGCG-3' (SEQ ID NO: 16). pUC19_CMVp-CAGp-EF1Ap was treated with the restriction enzymes (BsrGI and NheI). This fragment was ligated with the following UL8 fragment using In-Fusion to construct pUC19_CAGp-UL8 (#1.2). The UL8 fragment was prepared using the UL8 gene sequence (ATCC VR-1493D) as a template and the following primers: 5'-gaaggcacagtgtacaTCAGGCAAACAGAAACGACATCTTGTCGTC-3' (SEQ ID NO: 17) and 5'-caaagaattgGCTAGCATGGACACCGCAGATATCGTGTGGGTGGAG-3' (SEQ ID NO: 18). pUC19_CMVp-CAGp-EF1Ap was treated with the restriction enzymes (AscI and KpnI). This fragment was ligated with the following UL52 fragment using In-Fusion to construct pUC19_EF1Ap-UL52 (#1.3). The UL52 fragment was prepared using the UL52 gene sequence (ATCC VR-1493D) as a template and the following primers: 5'-gtgtcgtgaGGCGCGCCATGGGGCAGGAAGACGGGAACCGCGGGGAG-3' (SEQ ID NO: 19) and 5'-gccacccGAAGAGCggtaccTCAAGACGACGGTTGAGAGGTGCTGCAGGGAACGGACGGC-3' (SEQ ID NO: 20). pUC19_CMVp-UL5 and pUC19_EF1Ap-UL52 were treated with the restriction enzymes (AscI and NsiI) and ligated. The resulting plasmid and pUC19_CAGp-UL8 were treated with the restriction enzymes (BsrGI and XbaI) and ligated.

### • #2 SYNp149_UL29.30.42

pUC19_CMVp-CAGp-EF1Ap was treated with the restriction enzymes (SbfI and EcoRI). This fragment was ligated with the following UL42 fragment using In-Fusion to construct pUC19_CMVp-UL42 (#2.1). The UL42 fragment was prepared using the UL42 gene sequence (ATCC VR-1493D) as a template and the following primers: 5'-agagctGAAGAGCCCTGCAGGATGACGGATTCCCCTGGCGGTGTGGCCCCC-3' (SEQ ID NO: 21) and 5'-atgtatcttaGAATTCTCAGGGGAATCCAAAACCATACGGGGTTTG-3' (SEQ ID NO: 22). pUC19_CMVp-CAGp-EF1Ap was treated with the restriction enzymes (BsrGI and NheI). This fragment was ligated with the following UL29 fragment using In-Fusion to construct pUC19_CAGp-UL29 (#2.2). The UL29 fragment was prepared using the UL29 gene sequence (ATCC VR-1493D) as a template and the following primers: 5'-gaaggcacagtgtacaTCACAGCATATCCAACGTCAGGTCTCCCTTTTTG-3' (SEQ ID NO: 23) and 5'-caaagaattgGCTAGCATGGAGACAAAGCCCAAGACGGCAACCACC-3' (SEQ ID NO: 24). pUC19_CMVp-CAGp-EF1Ap was treated with the restriction enzymes (AscI and KpnI). This fragment was ligated with the following UL30 fragment using In-Fusion to construct pUC19_EF1Ap-UL30 (#2.3). The UL30 fragment was prepared using the UL30 gene sequence (ATCC VR-1493D) as a template and the following primers: 5'-gtgtcgtgaGGCGCGCCATGTTTTCCGGTGGCGGCGGCCCGCTGTCCCCCGGAGG-3' (SEQ ID NO: 25) and 5'-gccacccGAAGAGCggtaccTCATGCTAGAGTATCAAAGGCTCTATGCAAC-3' (SEQ ID NO: 26). pUC19_CMVp-UL42 and pUC19_CAGp-UL29 were treated with the restriction enzymes (HindIII and EcoRI) and ligated. The resulting plasmid and pUC19_EF1Ap-UL30 were treated with the restriction enzymes (AvrII and NsiI) and ligated.

### • #3 SYNp150_UL70.102.105

pUC19_CMVp-CAGp-EF1Ap was treated with the restriction enzymes (SbfI and EcoRI). This fragment was ligated with the following UL105 fragment using In-Fusion to construct pUC19_CMVp-UL105 (#3.1). The UL105 fragment was prepared using the UL105 gene sequence (ATCC VR-977D) as a template and the following primers: 5'-agagctGAAGAGCCCTGCAGGATGTCGATGACGGCCTCGTCATCCACACCGCGGCC-3' (SEQ ID NO: 27) and 5'-atgtatcttaGAATTCTCAAAAAATAAGCGTGGTGCGTTTGTCTTTG-3' (SEQ ID NO: 28). pUC19_CMVp-CAGp-EF1Ap was treated with the restriction enzymes (BsrGI and NheI). This fragment was ligated with the following UL102 fragment using In-Fusion to construct pUC19_CAGp-UL102 (#3.2). The UL102 fragment was prepared using the UL102 gene sequence (ATCC VR-977D) as a template and the following primers: 5'-gaaggcacagtgtacaTTAAGCGTTGAGCCGGAAAAACCGCAGGCC-3' (SEQ ID NO: 29) and 5'-caaagaattgGCTAGCATGACCGCTCAGCCGCCGCTGCACCACCGC-3' (SEQ ID NO: 30). pUC19_CMVp-CAGp-EF1Ap was treated with the restriction enzymes (AscI and KpnI). This fragment was ligated with the following UL70 fragment using In-Fusion to construct pUC19_EF1Ap-UL70 (#3.3). The UL70 fragment was prepared using the UL70 gene sequence (ATCC VR-977D) as a template and the following primers: 5'-gtgtcgtgaGGCGCGCCATGACGCTCGTTCTGTTCGCAACAGAGTATG-3' (SEQ ID NO: 31) and 5'-gccacccGAAGAGCggtaccTCAGACGGCGGTCGCCGGCGGCATGGGCGC-3' (SEQ ID NO: 32). pUC19_CMVp-UL105 and pUC19_CAGp-UL102 were treated with the restriction enzymes (SbfI and EcoRI) and ligated. The resulting plasmid and pUC19_EF1Ap-UL70 were treated with the restriction enzymes (AvrII and HindIII) and ligated.

### #4 SYNp151_UL44.54.57

pUC19_CMVp-CAGp-EF1Ap was treated with the restriction enzymes (SbfI and EcoRI). This fragment was ligated with the following UL44 fragment using In-Fusion to construct pUC19_CMVp-UL44 (#4.1). The UL44 fragment was prepared using the UL44 gene sequence (ATCC VR-977D) as a template and the following primers: 5'-agagctGAAGAGCCCTGCAGGATGGATCGCAAGACGCGCCTCTCGGAGCCAC-3' (SEQ ID NO: 33) and 5'-atgtatcttaGAATTCCTAGCCGCACTTTTGCTTCTTGGTGTTAGG-3' (SEQ ID NO: 34). pUC19_CMVp-CAGp-EF1Ap was treated with the restriction enzymes (BsrGI and NheI). This fragment was ligated with the following UL57 fragment using In-Fusion to construct pUC19_CAGp-UL57 (#4.2). The UL57 fragment was prepared using the UL57 gene sequence (ATCC VR-977D) as a template and the following primers: 5'-gaaggcacagtgtacaTTACAACCGGCTGCGTTTGGCCGGCAATAAC-3' (SEQ ID NO: 35) and 5'-caaagaattgGCTAGCATGAGCCACGAGGAACTAACCGCGCTAGCG-3' (SEQ ID NO: 36). pUC19_CMVp-CAGp-EF1Ap was treated with the restriction enzymes (AscI and KpnI). This fragment was ligated with the following UL54 fragment using In-Fusion to construct pUC19_EF1Ap-UL54 (#4.3). The UL54 fragment was prepared using the UL54 gene sequence (ATCC VR-977D) as a template and the following primers: 5'- gtgtcgtgaGGCGCGCCATGTTTTTCAACCCGTATCTGAGCGGCGGCGTGACCGG-3' (SEQ ID NO: 37) and 5'- gccacccGAAGAGCggtaccTCAACAGCATTCGTGCGCCTTGACACTGTAC-3' (SEQ ID NO: 38). pUC19_CMVp-UL44 and pUC19_CAGp-UL57 were treated with the restriction enzymes (SbfI and BamHI) and ligated. The resulting plasmid and pUC19_EF1Ap-UL54 were treated with the restriction enzymes (AvrII and HindIII) and ligated.

### · #5 SYNp152_UL112

The pUC19 vector plasmid was treated with the restriction enzymes (AatII and PciI). The following fragment was inserted therein using In-Fusion. This fragment was prepared using pUC19_CMVp-CAGp-EF1Ap as a template and the following primers: 5'- cgaaaagtgccacctgcggccgcaagctttctagaactagtcctaggggctccggtgcccg tcagtg-3' (SEQ ID NO: 39) and 5'- gctggccttttgctcgcggccgcagatctacgcgtgtcga-3' (SEQ ID NO: 40). The resulting plasmid was treated with the restriction enzyme (KpnI). This fragment was ligated with the following UL112 fragment using In-Fusion. The UL112 fragment was prepared using the UL112 gene sequence (ATCC VR-977D) as a template and the following primers: 5'- atccgaattcgctagcATGGATCTGCCTACTACCGTCGTGCGAAAATAC-3' (SEQ ID NO: 41) and 5'- gccacccGAAGAGCggtaccTTAATCGTCGAAAAACGCCGCGATCGAGGCGGCGGCGATC-3' (SEQ ID NO: 42).

### #6 pHelper

The pUC19 vector plasmid (Addgene: #500005) was treated with the restriction enzymes (AatII and PciI). The following three fragments (a VA fragment, an E4 fragment, and an E2A fragment) were inserted therein using In-Fusion. The VA fragment was prepared using the Ad5 genome (see ATCC VR-1516) as a template and the following primers: 5'- ttagtaagcttccctcctgacgcggtaggaggaggggagggtgccctgcatg-3' (SEQ ID NO: 43) and 5'- ccttttgctcacatgtcaattggtagatgtacctggacatccaggtgatgccggcg-3' (SEQ ID NO: 44). The E4 fragment was prepared using the Ad5 genome as a template and the following primers: 5'- tgtacaGGCGCGCCgaattcgttttagggcggagtaacttgtatgtgttgggaattgtag-3' (SEQ ID NO: 45) and 5'- agggaagcttactaaacggtacacaggaaacaggagacacaactccaagtg-3' (SEQ ID NO: 46). The E2A fragment was prepared using the Ad5 genome as a template and the following primers: 5'- gaaaagtgccacctgacgtcgcggccgcGCGATCGCggtacccaactccatgctcaacagt ccccaggtacagccc-3' (SEQ ID NO: 47) and 5'- gaattcGGCGCGCCtgtacagcccgggcgaccgcaccctgtgacgaaagccgcccgcaag-3' (SEQ ID NO: 48).

### #7 pR2C1

The pUC19 vector plasmid was treated with the restriction enzymes (AatII and PciI). The following three fragments (a p5 gene fragment, an AAV2 Rep fragment, and an SV40 ori fragment) were inserted therein using In-Fusion. The p5 gene fragment was prepared using the following primers: 5'- gaaaagtgccacctgacgtcgcggccgcggaggggtggagtcgtgacgtgaattacgtcat agggttagggaggtcctgtattagaggtcacgtgagtgttttgcgac-3' (SEQ ID NO: 49) and 5'- gttcaaacctcccgcttcaaaatggagaccctgcgtgctcactcgggcttaaatacccagc gtgaccacatggtgtcgcaaaatgtcgcaaaacactca-3' (SEQ ID NO: 50). The AAV2 Rep fragment was prepared using pRC2-mi342 (Takara) as a template and the following primers: 5'- gcgggaggtttgaacgcgcagccgccATGCCGGGGTTTTAC-3' (SEQ ID NO: 51) and 5'-GATCGCAGCGCTatttaaatcatTTATTGTTCAAAGAT-3' (SEQ ID NO: 52). The SV40 ori fragment was prepared using the SV40 ori gene sequence (TOYOBO) as a template and the following primers: 5'-aatAGCGCTGCGATCGCggcctccaaaaaagc-3' (SEQ ID NO: 53) and 5'- ccttttgctcacatgtcaattgatcccgcccctaact-3' (SEQ ID NO: 54). The resulting plasmid was treated with the restriction enzymes (SwaI and AfeI). An AAV1 Cap fragment was inserted therein using In-Fusion. The AAV1 Cap fragment was prepared using pRC1 (Takara) as a template and the following primers: 5'-AACAATAAatgatttaaatcagg-3' (SEQ ID NO: 55) and 5'- ggccGCGATCGCAGCGCTgtagccatggaaactagata-3' (SEQ ID NO: 56).

### · #8 pAAV-EGFP

(pAAV[Exp]-CMV>EGFP: WPRE) purchased from VectorBuilder.

### · #9 SYNp180-1

SYNp177_AIO_Helper-Ad5_R2C1_EGFP_wtE1_km was produced by gene synthesis and the OGAB method. The genes contained in this plasmid and their order are as follows: 5' ITR2 (VectorBuilder), CMV promoter (VectorBuilder), EGFP (VectorBuilder), WPRE (VectorBuilder), hGH polyA signal (TAKARA), 3' ITR2 (VectorBuilder), E2A (Ad5), E4 (Ad5), VA (Ad5), Cap (AAV1), Rep (AAV2), p5 (AAV2), E1A (Ad5), E1B (Ad5), pUC ori (pUC19), kanamycin resistance gene (VectorBuilder), HSV TK polyA signal (TAKARA), puromycin resistance gene (VectorBuilder), and mPGK promoter (VectorBuilder). Here, an exemplary procedure of the OGAB method is shown below. The nucleic acid of interest is divided into fragments of about 1 kb or less. The ends of each fragment are designed to be unique and complementary only to a specific fragment. Primers are designed for each of the fragments, and each fragment is prepared by PCR according to standard methods. Each fragment is cloned into a vector plasmid, and transformation of E. coli is performed using each vector plasmid to obtain a clone containing the correct sequence for each fragment. Plasmids are purified from these E. coli clones, and the DNA concentration of each plasmid solution is measured. Each plasmid solution is aliquoted and mixed so that the amounts of plasmids for each fragment are equal. A restriction enzyme is added to the mixed solution to generate DNA fragments having ends that are unique and complementary only to specific fragments. Thereafter, a quantity of an OGAB assembly vector cleaved with the same restriction enzyme is added to the solution so that its molar concentration matches that of each fragment, and ligation is performed to link pairs of fragments (including fragments of the OGAB assembly vector) having complementary ends. This is added to Bacillus subtilis competent cells, and after culturing, transformed colonies are obtained. The plasmid having the desired structure is recovered from the colony. SYNp177_AIO_Helper-Ad5_R2C1_EGFP_wtE1_km was treated with the restriction enzymes (FseI and SalI). SYNp180-1 was produced by inserting a p5/AAV2 Rep fragment into the site from which the E1/p5/AAV2 Rep fragment had been removed, using In-Fusion. The p5/AAV2 Rep fragment was prepared using plasmid #6, SYNp21_pR2C1, as a template and the following primers: 5'-TCACCATCTTGTCGACACAGTCGTTGAAGGG-3' (SEQ ID NO: 57) and 5'- agcgcagcgagtcGGCCGGCCgcggccgcggaggggtggagtcg-3' (SEQ ID NO: 58).

### · #10 SYNp162_pGETS151_AIO-R2C2-EGFP

It was produced by gene synthesis and the OGAB method. The genes contained in this plasmid and their order are as follows: 5' ITR2 (TAKARA), CMV promoter (TAKARA), EGFP (VectorBuilder), WPRE (VectorBuilder), hGH polyA signal (TAKARA), 3' ITR2 (TAKARA), E2A (Ad2), E4 (Ad2), VA (Ad2), Rep (AAV2), Cap (AAV2), CMV promoter (TAKARA), mi342 (TAKARA), HSV TK polyA signal (TAKARA), p5 (TAKARA), tetL (pGETS151), Bacillus subtilis origin of replication (pGETS151), Escherichia coli origin of replication (pGETS151), and ampicillin resistance gene (pGETS151). An exemplary procedure of the OGAB method is described above.

### · #11 SYNp163_pGETS151_AIO-R2C3-EGFP

Plasmid #10, SYNp162_pGETS151_AIO-R2C2-EGFP, was treated with the restriction enzyme SgrDI. A fragment prepared using the Rep2-Cap3 gene sequence as a template and the following primers was inserted therein using In-Fusion: 5'-ACATCTACGGATTACGTCGAggaggggtggagtcgtgacg-3' (SEQ ID NO: 59) and 5'-ACCGCATTAAAGCTCGTCGAAGCGCTgtagccatggaaac-3' (SEQ ID NO: 60).

### · #12 SYNp164_pGETS151_AIO-R2C4-EGFP

Plasmid #10, SYNp162_pGETS151_AIO-R2C2-EGFP, was treated with the restriction enzyme SgrDI. A fragment prepared using the Rep2-Cap4 gene sequence as a template and primers of SEQ ID NO: 59 and SEQ ID NO: 60 was inserted therein using In-Fusion.

### · #13 SYNp165_pGETS151_AIO-R2C5-EGFP

Plasmid #10, SYNp162_pGETS151_AIO-R2C2-EGFP, was treated with the restriction enzyme SgrDI. A fragment prepared using the Rep2-Cap5 gene sequence as a template and primers of SEQ ID NO: 59 and SEQ ID NO: 60 was inserted therein using In-Fusion.

### · #14 SYNp166_pGETS151_AIO-R2C6-EGFP

Plasmid #10, SYNp162_pGETS151_AIO-R2C2-EGFP, was treated with the restriction enzyme SgrDI. A fragment prepared using the Rep2-Cap6 gene sequence as a template and primers of SEQ ID NO: 57 and SEQ ID NO: 58 was inserted therein using In-Fusion.

### · #15 SYNp167_pGETS151_AIO-R2C7-EGFP

Plasmid #10, SYNp162_pGETS151_AIO-R2C2-EGFP, was treated with the restriction enzyme SgrDI. A fragment prepared using the Rep2-Cap7 gene sequence as a template and primers of SEQ ID NO: 59 and SEQ ID NO: 60 was inserted therein using In-Fusion.

### · #16 SYNp168_pGETS151_AIO-R2C8-EGFP

Plasmid #10, SYNp162_pGETS151_AIO-R2C2-EGFP, was treated with the restriction enzyme SgrDI. A fragment prepared using the Rep2-Cap8 gene sequence as a template and primers of SEQ ID NO: 59 and SEQ ID NO: 60 was inserted therein using In-Fusion.

· Plasmid #17, SYNp169_pGETS151_AIO-R2C9-EGFP, was prepared by treating plasmid #10, SYNp162_pGETS151_AIO-R2C2-EGFP, with the restriction enzyme SgrDI. A fragment prepared using the Rep2-Cap9 gene sequence as a template and primers of SEQ ID NO: 59 and SEQ ID NO: 60 was inserted therein using In-Fusion.

### · #18 SYNp170_pGETS151_AIO-R2C10-EGFP

Plasmid #10, SYNp162_pGETS151_AIO-R2C2-EGFP, was treated with the restriction enzyme SgrDI. A fragment prepared using the Rep2-Cap10 gene sequence as a template and primers of SEQ ID NO: 59 and SEQ ID NO: 60 was inserted therein using In-Fusion.

### · #19 SYNp180-3

Plasmid #9, SYNp180-1, was treated with the restriction enzymes (SalI and AsiSI). A fragment prepared using the Rep2-Cap3 gene sequence as a template and the following primers was inserted therein using In-Fusion: 5'- gtgagcaaaaggGCGATCGCTCTAGAACTAGTgtagccatggaaactagata-3' (SEQ ID NO: 61) and 5'-CAACGACTGTGTCGACAAGATG-3' (SEQ ID NO: 62).

### · #20 pUC19_CMV-Ad5Pol

The pUC19 vector plasmid was treated with the restriction enzymes (AatII and PciI). The following two fragments (a CMV promoter fragment and an hGH polyA fragment) were inserted therein using In-Fusion. The CMV promoter fragment was prepared using the CMV promoter gene sequence (VectorBuilder) as a template and the following primers: 5'- gaaaagtgccacctgcggccgcaagctttctaggcgttacataacttacggtaa-3' (SEQ ID NO: 63) and 5'- ggtaccGGCGCGCCggatccGCTCTTCagctctgcttatatagacctc-3' (SEQ ID NO: 64). The hGH polyA fragment was prepared using the hGH polyA signal gene sequence (Takara) as a template and the following primers: 5'- ggtaccGGCGCGCCggatccGCTCTTCagctctgcttatatagacctc-3' (SEQ ID NO: 65) and 5'- gctggccttttgctcgcggccgcagatctacgcgtgtcgacaaggacagggaagggagcag -3' (SEQ ID NO: 66). This plasmid was treated with the restriction enzymes (BamHI and AscI). An Ad5-derived polymerase gene fragment was inserted therein using In-Fusion. The Ad5 polymerase gene fragment was prepared using Ad5 genomic extract as a template and the following primers: 5'- gctGAAGAGCggatccgccaccatggacagctcccatctgcgcgatgttgtcatcaagctc c-3' (SEQ ID NO: 67) and 5'-AGCggtaccGGCGCGCCtacggcatctcgatccagcatatctcctcgtttcgcgggttg-3' (SEQ ID NO: 68).

In particular, an overview of the structure of the plasmid encoding the human herpesvirus (HHV)-derived gene examined in the present example is summarized below.

**[Table 1]**

| **HHV Plasmid (full length)** | **Gene** | **Gene length (bp)** | **Function** | **Origin of the virus** | **Promoter** | **PolyA signal** |
|---|---|---|---|---|---|---|
| #1 **SYNp148** (14223 bp) | UL8 | 2253 | Helicase-primase subunit | HHV-1 | CAG | bGH |
| | UL5 | 2649 | Helicase subunit of helicase-primase | HHV-1 | CMV | SV40 |
| | UL52 | 3177 | Primase subunit of helicase-primase | HHV-1 | EF1A | hGH |
| #2 SYNp149 (14910 bp) | UL29 (ICP8) | 3591 | Single-stranded DNA binding protein | HHV-1 | CAG | bGH |
| | UL30 | 3708 | DNA polymerase catalytic subunit | HHV-1 | EF1A | hGH |
| | UL42 | 1467 | DNA polymerase processivity subunit | HHV-1 | CMV | SV40 |
| #3 SYNp150 (14478 bp) | UL102 | 2622 | Helicase-primase subunit | HHV-5 | CAG | bGH |
| | UL105 | 2871 | Helicase subunit of helicase-primase | HHV-5 | CMV | SV40 |
| | UL70 | 2841 | Primase subunit of helicase-primase | HHV-5 | EF1A | hGH |
| #4 SYNp151 (14883 bp) | UL57 | 3708 | Single-stranded DNA binding protein | HHV-5 | CAG | bGH |
| | UL54 | 3729 | DNA polymerase catalytic subunit | HHV-5 | EF1A | hGH |
| | UL44 | 1302 | DNA polymerase processivity subunit | HHV-5 | CMV | SV40 |
| #5 SYNp152 (5753 bp) | UL112 | 2203 | Gene regulation, DNA replication | HHV-5 | EF1A | hGH |

### (Example 1: AAV production using human herpesvirus (HHV)-derived gene)

The production of AAV1 vectors in multiple cell lines was examined using genes associated with replication of the HHV genes as alternatives to the helper genes of adenovirus of serotype 5 (Ad5).

### Production of rAAV

On the day before transfection, 5 x 10⁶ HEK293 cells, HeLa cells, or CHO-K1 cells were seeded into a 6-well plate. After 22 hours, the medium was replaced with 1.9 mL of medium (2% FBS and 1% penicillin/streptomycin), and the cells were cultured at 37°C and 5% CO₂ for 2 hours. To the confluent cells, a transfection solution was added, the transfection solution being prepared by mixing 50 µL of DMEM medium containing the plasmids listed in the table below with 50 µL of DMEM medium containing PEIpro (registered trademark) (PolyPlus-transfection SAS) at an amount equal to the amount of DNA.

**[Table 2]**

| pDNA# | Transfected pDNA | Length [bp] | Amount of transfected pDNA |
|---|---|---|---|
| | | | [µg/well] |
| 1 | SYNp148_UL5.8.52 | 14223 | 0.66 |
| 2 | SYNp149_UL29.30.42 | 14910 | 0.69 |
| 3 | SYNp 150_UL70.102.105 | 14478 | 0.67 |
| 4 | SYNp151_UL44.54.57 | 14883 | 0.69 |
| 6 | pHelper | 11120 | 0.52 |
| 7 | pR2C1 | 6336 | 0.30 |
| 8 | pAAV-EGFP | 5118 | 0.24 |

### · Evaluation of rAAV

After culturing at 37°C and 5% CO₂ for 72 hours, in order to quantify the genome titer (copy number) of rAAV contained in the supernatant, the supernatant was treated with DNase I for 30 minutes, and then, the sample was reacted at 95°C for 10 minutes, diluted 1,000-fold, and subjected to qPCR. Primer sequences 5'-CATCAATGGGCGTGGATAGC-3' (SEQ ID NO: 1) and 5'-GGAGTTGTTACGACATTTTGGAAA-3' (SEQ ID NO: 2) were used to target the CMV promoter. The PCR conditions were as follows: an initial step at 95°C for 10 minutes, followed by 40 cycles of 95°C for 15 seconds, 55°C for 5 seconds, and 72°C for 30 seconds.

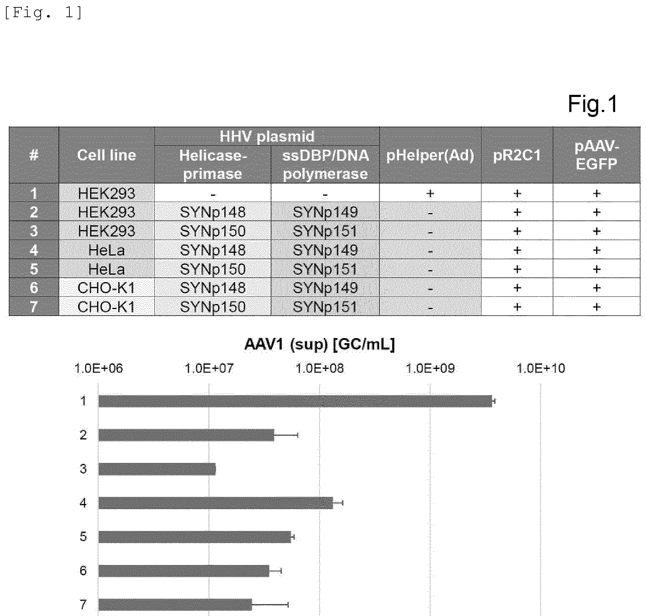

The results are shown in FIG. 1. It was confirmed that AAV vectors were produced even when genes associated with replication of herpesvirus genes were used instead of adenovirus helper genes. Note that HEK293 cells contain the adenovirus helper genes E1A and E1B, whereas HeLa cells and CHO-K1 cells do not contain these helper genes. Nevertheless, since production of AAV vectors was observed even in HeLa cells and CHO-K1 cells to which E1A and E1B were not added, genes associated with replication of the herpesvirus genes may be useful for the production of AAV vectors.

### (Example 2: Effect of herpesvirus genes on yield of AAV vectors when used as supplements to adenovirus helper genes)

The yield of AAV1 vectors in HEK293 cells was examined when genes associated with replication of HHV genes were used in addition to the helper genes of Ad5.

### Production of rAAV

On the day before transfection, 5 x 10⁶ HEK293 cells were seeded into a 6-well plate. After 22 hours, the medium was replaced with 1.9 mL of DMEM medium (2% FBS and 1% penicillin/streptomycin), and the cells were cultured at 37°C and 5% CO₂ for 2 hours. To the confluent cells, a transfection solution was added, the transfection solution being prepared by mixing 50 µL of DMEM medium containing the plasmids listed in the table below with 50 µL of DMEM medium containing PEIpro (registered trademark) (PolyPlus-transfection SAS) at an amount equal to the amount of DNA.

**[Table 3]**

| pDNA# | Transfected pDNA | Length [bp] | Amount of transfected pDNA |
|---|---|---|---|
| | | | [µg/well] |
| 1 | SYNp148_UL5.8.52 | 14223 | 0.66 |
| 2 | SYNp149_UL29.30.42 | 14910 | 0.69 |
| 3 | SYNp150_UL70.102.105 | 14478 | 0.67 |
| 4 | SYNp151_UL44.54.57 | 14883 | 0.69 |
| 6 | pHelper | 11120 | 0.52 |
| 7 | pR2C1 | 6336 | 0.30 |
| 8 | pAAV-EGFP | 5118 | 0.24 |
| 9 | SYNp180-1 | 19464 | 0.91 |

### · Evaluation of rAAV

After culturing for 72 hours at 37°C and 5% CO₂, in order to quantify the genome titer of rAAV contained in the supernatant, the supernatant was treated with DNase I for 30 minutes, and then, the sample was reacted at 95°C for 10 minutes, diluted 1,000-fold, and subjected to qPCR. Primer sequences 5'-CATCAATGGGCGTGGATAGC-3' (SEQ ID NO: 1) and 5'-GGAGTTGTTACGACATTTTGGAAA-3' (SEQ ID NO: 2) were used to target the CMV promoter. The PCR conditions were as follows: an initial step at 95°C for 10 minutes, followed by 40 cycles of 95°C for 15 seconds, 55°C for 5 seconds, and 72°C for 30 seconds.

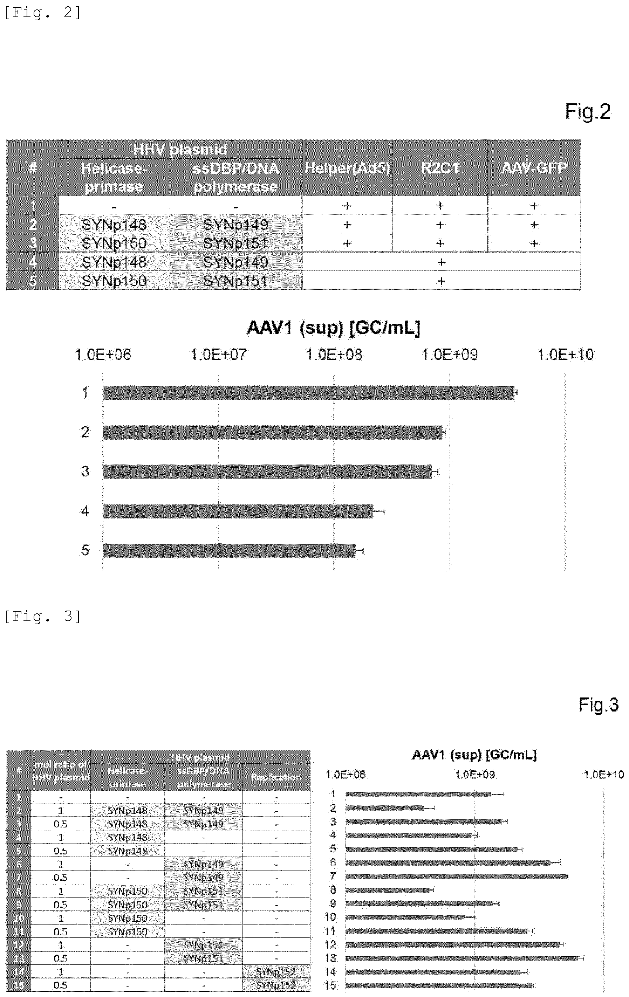

The results are shown in FIG. 2. The yield of the AAV vectors was increased by adding the adenovirus helper genes rather than a gene alone associated with replication of a herpesvirus gene.

### (Example 3: Effect on yield of AAV vectors by selection of herpesvirus gene)

The yield of AAV1 vectors in HEK293 cells was examined when HHV genes were used in addition to Ad5 helper genes, by varying the types and amounts of genes associated with replication of HHV genes.

### Production of rAAV

On the day before transfection, 5 x 10⁶ HEK293 cells were seeded into a 6-well plate. After 22 hours, the medium was replaced with 1.9 mL of DMEM medium (2% FBS and 1% penicillin/streptomycin), and the cells were cultured at 37°C and 5% CO₂ for 2 hours. To the confluent cells, a transfection solution was added, the transfection solution being prepared by mixing 50µL of DMEM medium containing the plasmids listed in the table below with 50 µL of DMEM medium containing PEIpro (registered trademark) (PolyPlus-transfection SAS) at an amount equal to the amount of DNA.

**[Table 4]**

| pDNA# | Transfected pDNA | Length [bp] | Amount of transfected pDNA (molar ratio = 1.0) | Amount of transfected pDNA (molar ratio = 0.5) |
|---|---|---|---|---|
| | | | [µg/well] | [µg/well] |
| 1 | SYNp148_UL5.8.52 | 14223 | 0.66 | 0.33 |
| 2 | SYNp149_UL29.30.42 | 14910 | 0.69 | 0.35 |
| 3 | SYNp150_UL70.102.105 | 14478 | 0.67 | 0.34 |
| 4 | SYNp151_UL44.54.57 | 14883 | 0.69 | 0.35 |
| 5 | SYNp152_UL112 | 5753 | 0.27 | 0.13 |
| 9 | SYNp180-1 | 19464 | 0.91 | - |

### · Evaluation of rAAV

After culturing for 72 hours at 37°C and 5% CO₂, in order to quantify the genome titer of rAAV contained in the supernatant, the supernatant was treated with DNase I for 30 minutes, and then, the sample was reacted at 95°C for 10 minutes, diluted 1,000-fold, and subjected to qPCR. Primer sequences 5'-CATCAATGGGCGTGGATAGC-3' (SEQ ID NO: 1) and 5'-GGAGTTGTTACGACATTTTGGAAA-3' (SEQ ID NO: 2) were used to target the CMV promoter. The PCR conditions were as follows: an initial step at 95°C for 10 minutes, followed by 40 cycles of 95°C for 15 seconds, 55°C for 5 seconds, and 72°C for 30 seconds.

The results are shown in FIG. 3. Here, a helicase, which unwinds the double-stranded DNA, and a primase, which has primer addition activity, were introduced together as a gene having continuous functions (a helicase-primase gene). In addition, a DNA-binding protein and a polymerase were introduced together as a gene having continuous functions (a DNA strand synthesis gene). In particular, a high yield of AAV vectors was obtained when the DNA strand synthesis gene was introduced. It was found that a higher yield of AAV vectors can be obtained when the helicase-primase gene and the DNA strand synthesis gene are introduced alone than when the helicase-primase gene and the DNA strand synthesis gene are introduced in combination, and a higher yield of AAV vectors can be obtained than when the HHV gene is not introduced. In addition, similar results were obtained by introducing UL112, which is a gene associated with replication of another gene, alone. The yield of AAV vectors was generally higher when the molar ratio of the herpes gene to the helper gene introduced into the producer cell was 0.5, compared to when the molar ratio was 1. As a result of further examining the molar ratio by another experiment, the effect of the addition of the herpes gene was confirmed even at a molar ratio of 0.05. Note that, in order to reduce the number of types of plasmids to be transfected and simplify the experiment, the experiment was performed using plasmids (SYNp148, SYNp149, and the like) containing multiple HHV genes, but similar results can be obtained even when the genes are divided into separate plasmids.

### (Example 4: Effect of introduction of herpesvirus genes on yield of AAV vectors for each serotype of AAV capsid)

The yield of AAV vectors in HEK293 cells was examined by varying the serotype of the AAV capsid to be introduced and, in addition to Ad5 helper genes, incorporating genes associated with replication of HHV genes.

### Production of rAAV

On the day before transfection, 5 x 10⁶ HEK293 cells were seeded into a 6-well plate. After 22 hours, the medium was replaced with 1.9 mL of DMEM medium (2% FBS and 1% penicillin/streptomycin), and the cells were cultured at 37°C and 5% CO₂ for 2 hours. To the confluent cells, a transfection solution was added, the transfection solution being prepared by mixing 50 µL of DMEM medium containing the plasmids listed in the table below with 50 µL of DMEM medium containing PEIpro (registered trademark) (PolyPlus-transfection SAS) at an amount equal to the amount of DNA.

**[Table 5]**

| pDNA# | Transfected pDNA | Length [bp] | Amount of transfected pDNA |
|---|---|---|---|
| | | | [µg/well] |
| 2 | SYNp149_UL29.30.42 | 14910 | 0.69 |
| 4 | SYNp151_UL44.54.57 | 14883 | 0.69 |
| 5 | SYNp152_UL112 | 5753 | 0.27 |
| 9 | SYNp180-1 | 19464 | 0.91 |
| 10 | SYNp162_pGETS151_AIO-R2C2-EGFP | 23762 | 1.11 |
| 11 | SYNp163_pGETS151_AIO-R2C3-EGFP | 22724 | 1.06 |
| 12 | SYNp164_pGETS151_AIO-R2C4-EGFP | 22718 | 1.06 |
| 13 | SYNp165_pGETS151_AIO-R2C5-EGFP | 22688 | 1.06 |
| 14 | SYNp166_pGETS151_AIO-R2C6-EGFP | 22724 | 1.06 |
| 15 | SYNp167_pGETS151_AIO-R2C7-EGFP | 22727 | 1.06 |
| 16 | SYNp168_pGETS151_AIO-R2C8-EGFP | 22730 | 1.06 |
| 17 | SYNp169_pGETS151_AIO-R2C9-EGFP | 22724 | 1.06 |
| 18 | SYNp170_pGETS151_AIO-R2C10-EGFP | 22730 | 1.06 |

### · Evaluation of rAAV

After culturing for 72 hours at 37°C and 5% CO₂, in order to quantify the genome titer of rAAV contained in the supernatant, the supernatant was treated with DNase I for 30 minutes, and then, the sample was reacted at 95°C for 10 minutes, diluted 1,000-fold, and subjected to qPCR. Primer sequences 5'-CATCAATGGGCGTGGATAGC-3' (SEQ ID NO: 1) and 5'-GGAGTTGTTACGACATTTTGGAAA-3' (SEQ ID NO: 2) were used to target the CMV promoter. The PCR conditions were as follows: an initial step at 95°C for 10 minutes, followed by 40 cycles of 95°C for 15 seconds, 55°C for 5 seconds, and 72°C for 30 seconds.

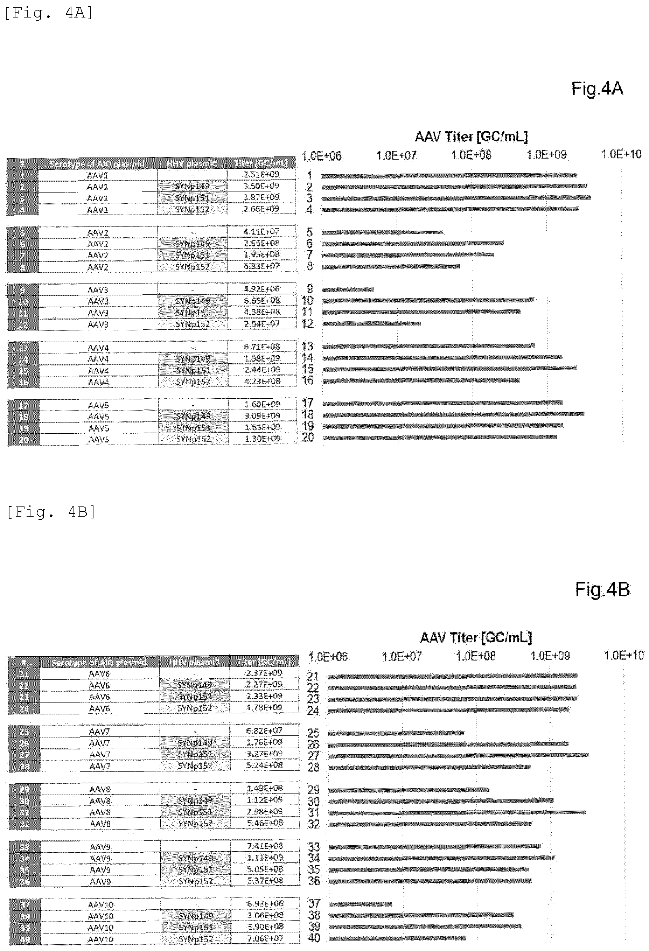

The results are shown in FIGS. 4A and 4B. As in Example 3, many cases, in which the use of the genes associated with replication of the HHV genes led to an increased yield of AAV vectors, were confirmed. In particular, the results of samples #6 and #7 (combinations of AAV2 and SYNp149 or SYNp151) have attracted attention, and although AAV2 is less likely to be secreted into the supernatant and is usually recovered from the cell extract, the present results showing an increase in the amount of AAV vectors in the supernatant suggest that it may be effective in terms of productivity.

### (Example 5: AAV vector production using single HHV gene)

The yield of AAV3 vectors in HEK293 cells was examined when each of the genes associated with replication of HHV shown in Table 6 was individually added to the Ad5 helper genes.

**[Table 6]**

| Origin of the virus | **Gene** | **Function** |
|---|---|---|
| HHV-1 | UL8 | Helicase-primase subunit |
| | UL5 | Helicase subunit of helicase-primase |
| | UL52 | Primase subunit of helicase-primase |
| | UL29 (ICP8) | Single-stranded DNA binding protein |
| | UL30 | DNA polymerase catalytic subunit |
| | UL42 | DNA polymerase processivity subunit |
| HHV-5 | UL102 | Helicase-primase subunit |
| | UL105 | Helicase subunit of helicase-primase |
| | UL70 | Primase subunit of helicase-primase |
| | UL57 | Single-stranded DNA binding protein |
| | UL54 | DNA polymerase catalytic subunit |
| | UL44 | DNA polymerase processivity subunit |

### · Production of rAAV

On the day before transfection, 0.225 × 10⁶ HEK293 cells were seeded into a 24-well plate. After 22 hours, the medium was replaced with 0.38 mL of DMEM medium (2% FBS and 1% penicillin/streptomycin), and the cells were cultured at 37°C and 5% CO₂ for 2 hours. To the confluent cells, a transfection solution was added, the transfection solution being prepared by mixing 10µL of DMEM medium in which each pDNA was added to pDNA#19 shown in the table below with 10 µL of DMEM medium containing PEIpro (registered trademark) (PolyPlus-transfection SAS) at an amount equal to the amount of DNA.

**[Table 6A]**

| pDNA# | Transfected pDNA | Length [bp] | Amount of transfected pDNA |
|---|---|---|---|
| | | | [µg/well] |
| 1.1 | pUC19_CMVp-UL5 | 8793 | 0.086 |
| 1.2 | pUC19_CAGp-UL8 | 8397 | 0.082 |
| 1.3 | pUC19_EF1Ap-UL52 | 9321 | 0.091 |
| 2.1 | pUC19_CMVp-UL42 | 7611 | 0.074 |
| 2.2 | pUC19_CAGp-UL29 | 9735 | 0.095 |
| 2.3 | pUC19_EF1Ap-UL30 | 9852 | 0.096 |
| 3.1 | pUC19_CMVp-UL105 | 9015 | 0.088 |
| 3.2 | pUC19_CAGp-UL102 | 8766 | 0.086 |
| 3.3 | pUC19_EF1Ap-UL70 | 8985 | 0.088 |
| 4.1 | pUC19_CMVp-UL44 | 7446 | 0.073 |
| 4.2 | pUC19_CAGp-UL57 | 9852 | 0.096 |
| 4.3 | pUC19_EF1Ap-UL54 | 9873 | 0.096 |
| 19 | SYNp180-3 | 19464 | 0.380 |

### · Evaluation of rAAV

After culturing for 72 hours at 37°C and 5% CO₂, in order to quantify the genome titer of rAAV contained in the supernatant, the supernatant was treated with DNase I for 30 minutes, and then, the sample was reacted at 95°C for 10 minutes, diluted 1,000-fold, and subjected to qPCR. Primer sequences 5'-CATCAATGGGCGTGGATAGC-3' (SEQ ID NO: 1) and 5'-GGAGTTGTTACGACATTTTGGAAA-3' (SEQ ID NO: 2) were used to target the CMV promoter. The PCR conditions were as follows: an initial step at 95°C for 10 minutes, followed by 40 cycles of 95°C for 15 seconds, 55°C for 5 seconds, and 72°C for 30 seconds.

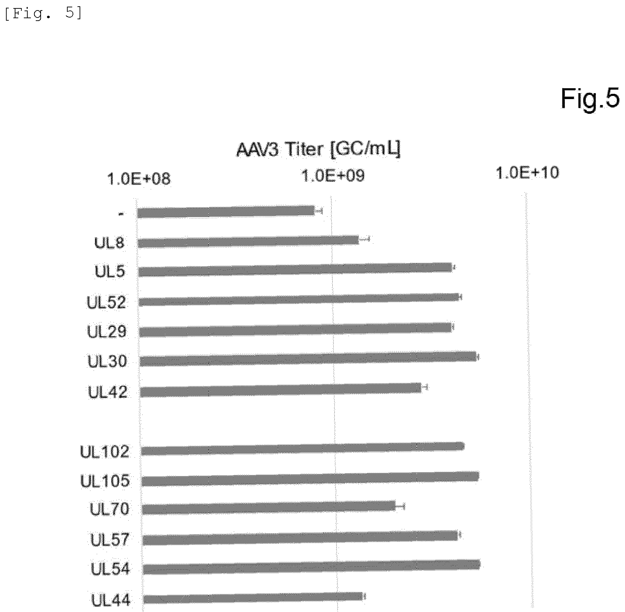

The results are shown in FIG. 5. When a gene associated with replication of HHV was added alone to the Ad helper genes, the yield of rAAV3 was increased for all such genes, as compared to the case where it is not added. In particular, high yields were commonly observed upon the addition of the DNA polymerase catalytic subunits represented by UL30 of HHV-1 and UL54 of HHV-5. Based on these results, the addition of genes associated with replication can increase the yield of rAAV vectors similarly in other HHV serotypes.

### (Example 6: AAV vector production using various HHV genes)

The yield of AAV vectors was confirmed when one or more genes associated with replication of various herpesviruses, as listed in the table below, are transfected alone or in combination with AAV production genes.

**[Table 7]**

| Origin of the virus | **Gene** | **Function** |
|---|---|---|
| HHV-2 | UL5 | Helicase subunit of helicase-primase |
| | UL8 | Helicase-primase subunit |
| | UL9 | DNA replication origin-binding helicase |
| | UL29 | Single-stranded DNA binding protein |
| | UL30 | DNA polymerase catalytic subunit |
| | UL42 | DNA polymerase processivity subunit |
| | UL52 | Primase subunit of helicase-primase |
| HHV-3 | ORF6 | Components of the DNA helicase-primase complex |
| | ORF16 | Subunits of DNA polymerase |
| | ORF28 | DNA polymerase catalytic subunit |
| | ORF29 | Single-stranded DNA binding protein |
| | ORF55 | Components of the DNA helicase-primase complex |
| HHV-4 | BMRF1 | DNA polymerase processivity subunit |
| | BSLF1 | Primase subunit of helicase-primase |
| | BBLF4 | Helicase subunit of helicase-primase |
| | BBLF2/BBLF3 | Helicase-primase subunit |
| | BALF5 | DNA polymerase catalytic subunit |
| | BALF2 | Single-stranded DNA binding protein |
| HHV-6A | U27 | Replication, double-stranded DNA binding protein, polymerase processivity subunit; transactivation |
| | U38 | DNA polymerase catalytic subunit |
| | U41 | Major DNA-binding proteins |
| HHV-6B | U38 | DNA polymerase |
| | U41 | Major DNA-binding proteins |
| | U79 | DNA polymerase |
| HHV-7 | U27 | DNA polymerase processivity subunit |
| | U38 | DNA polymerase catalytic subunit |
| | U41 | Single-stranded DNA binding protein |
| | U43 | Primase subunit of helicase-primase |
| | U73 | DNA replication origin-binding helicase |
| | U74 | Helicase-primase subunit |
| | U77 | Helicase subunit of helicase-primase |
| | U79 | Gene Regulation |
| HHV-8 | ORF6 | Single-stranded DNA binding protein |
| | ORF9 | DNA polymerase catalytic subunit |
| | ORF59 | DNA polymerase processivity subunit |

It can be confirmed that genes associated with replication of herpesvirus genes are capable of producing AAV vectors not only when introduced alone, but also when introduced in combination.

### (Example 7: AAV vector production using HHV polymerase alone as helper gene)

The yield of AAV vectors is confirmed when the plasmids shown in the table below (see Example 1) are transfected together with pR2C1 and pAAV-EGFP using HEK293, HeLa, and CHO-K1 cells without being combined with helper genes.

**[Table 8]**

| **Cell line** | **HHV Plasmids** | **Helper genes (Ad)** | **pR2C1** | **pAAV-EGFP** |
|---|---|---|---|---|
| HEK293 | - | + | + | + |
| HEK293 | SYNp148 | - | + | + |
| HEK293 | SYNp149 | - | + | + |
| HEK293 | SYNp150 | - | + | + |
| HEK293 | SYNp151 | - | + | + |
| HeLa | SYNp148 | - | + | + |
| HeLa | SYNp149 | - | + | + |
| HeLa | SYNp150 | - | + | + |
| HeLa | SYNp151 | - | + | + |
| CHO-K1 | SYNp148 | - | + | + |
| CHO-K1 | SYNp149 | - | + | + |
| CHO-K1 | SYNp150 | - | + | + |
| CHO-K1 | SYNp151 | - | + | + |

It is confirmed that the AAV vectors are produced, and a higher yield of the AAV vectors can be obtained compared to the case where helper genes are introduced.

### (Example 8: AAV vector production using various Ad DNA polymerase genes)

From seven species of adenoviruses A, B, C, D, E, F, and G, serotypes Ad12, Ad14, Ad5, Ad8, Ad4, Ad40, and Ad52 are respectively selected, and the yield of AAV vectors when the DNA polymerase gene is transfected together with AAV production gene is confirmed.

A higher yield of AAV vectors can be obtained when an adenovirus DNA polymerase is introduced, compared to the case where it is not introduced.

### (Example 9: AAV vector production using Ad DNA polymerase gene)

The yield of AAV3 vectors in HEK293 cells was confirmed when not only the herpesvirus-derived gene but also the adenovirus 5-derived DNA polymerase gene was added.

### Production of rAAV

On the day before transfection, 0.225 × 10⁶ HEK293 cells were seeded into a 24-well plate. After 22 hours, the medium was replaced with 0.38 mL of DMEM medium (2% FBS and 1% penicillin/streptomycin), and the cells were cultured at 37°C and 5% CO₂ for 2 hours. To the confluent cells, a transfection solution was added, the transfection solution being prepared by mixing 10 µL of DMEM medium containing the plasmids listed in the table below with 10 µL of DMEM medium containing PEIpro (registered trademark) (PolyPlus-transfection SAS) at an amount equal to the amount of DNA.

**[Table 9]**

| pDNA# | Transfected pDNA | Length [bp] | Amount of transfected pDNA |
|---|---|---|---|
| | | | [µg/well] |
| 20 | pUC19_CMV-Ad5Pol | 6046 | 0.059 |
| 19 | SYNp180-3 | 19464 | 0.380 |

### · Evaluation of rAAV

After culturing for 72 hours at 37°C and 5% CO₂, in order to quantify the genome titer of rAAV contained in the supernatant, the supernatant was treated with DNase I for 30 minutes, and then, the sample was reacted at 95°C for 10 minutes, diluted 1,000-fold, and subjected to qPCR. Primer sequences 5'-CATCAATGGGCGTGGATAGC-3' (SEQ ID NO: 1) and 5'-GGAGTTGTTACGACATTTTGGAAA-3' (SEQ ID NO: 2) were used to target the CMV promoter. The PCR conditions were as follows: an initial step at 95°C for 10 minutes, followed by 40 cycles of 95°C for 15 seconds, 55°C for 5 seconds, and 72°C for 30 seconds.

A higher yield of AAV vectors was confirmed when an adenovirus DNA polymerase was introduced, compared to the case where it was not introduced. Based on these results, the yield of rAAV vectors can be improved similarly in DNA polymerase genes of the serotypes of the seven species of adenoviruses A, B, C, D, E, F, and G.

### (Note)

As described above, although the present disclosure is exemplified by the use of preferred embodiments of the present disclosure, it is understood that the scope of the present disclosure should be interpreted solely based on the claims. It is also understood that any patent, any patent application, and any references cited herein should be incorporated herein by reference in the same manner as the contents are specifically described herein. The present application claims priority to Japanese Patent Application No. 2023-013329, filed on January 31, 2023 to the Japan Patent Office, the entire contents of which are incorporated herein by reference as necessary.

### Industrial Applicability

The present disclosure provides a virus-derived gene that can be used in the production of a novel virus-derived construct, thereby enabling improved provision of a virus-derived construct.

### Sequence Listing Free Text

SEQ ID NO: 1: CATCAATGGGCGTGGATAGC, Primer 1 for CMV promoter
· SEQ ID NO: 2: GGAGTTGTTACGACATTTTGGAAA, Primer 2 for CMV promoter
· SEQ ID NO: 3:
   TTAGTAAGCTTCCCTCCTGACGCGGTAGGAGGAGGGGAGGGTGCCCTGCATG, Primer 1 for CMV promoter fragment
· SEQ ID NO: 4:
   TTAGTAAGCTTCCCTCCTGACGCGGTAGGAGGAGGGGAGGGTGCCCTGCATG, Primer 2 for CMV promoter fragment
· SEQ ID NO: 5: CCCTGCAGGGAATTCTAAGATACATTGATGAG, Primer 1 for SV40 polyA fragment
· SEQ ID NO: 6: GGAGGATCCAACTTGTTTATTGCAGCTTATA, Primer 2 for SV40 polyA fragment
· SEQ ID NO: 7: CAAGTTGGATCCTCCCCAGCATGCCTGCTATTC, Primer 1 for bGH polyA fragment
· SEQ ID NO: 8: TTGGCTAGCTGTACACTGTGCCTTCTAGTTGCC, Primer 2 for bGH polyA fragment
· SEQ ID NO: 9: CAAGTTGGATCCTCCCCAGCATGCCTGCTATTC, Primer 1 for CAG promoter fragment
· SEQ ID NO: 10:
   GCTGGCCTTTTGCTCGCGGCCGCAGATCTACGCGTCCTAGGCTCGACATTGATTATTGAC, Primer 2 for CAG promoter fragment
· SEQ ID NO: 11: CAATGTCGAGCCTAGGGGCTCCGGTGCCCGTCAGTG, Primer 1 for EFA1 promoter fragment
· SEQ ID NO: 12:
   GAAGAGCGGTACCGGCGCGCCTCACGACACCTGAAATGGAA, Primer 2 for EFA1 promoter fragment
· SEQ ID NO: 13: CCGGTACCGCTCTTCGGGTGGCATCCCTGTGACCC, Primer 1 for hGH polyA fragment
· SEQ ID NO: 14: TCTACGCGTGTCGACAAGGACAGGGAAGGGAGCAG, Primer 2 for hGH polyA fragment
· SEQ ID NO: 15:
   AGAGCTGAAGAGCCCTGCAGGATGGCGGCGGCCGGCGGGGAGCGCCAGCTAG, Primer 1 for UL5 fragment
· SEQ ID NO: 16:
   ATGTATCTTAGAATTCTTAATAGACAATGACCACGTTCGGATCGCG, Primer 2 for UL5 fragment
· SEQ ID NO: 17:
   GAAGGCACAGTGTACATCAGGCAAACAGAAACGACATCTTGTCGTC, Primer 1 for UL8 fragment
· SEQ ID NO: 18:
   CAAAGAATTGGCTAGCATGGACACCGCAGATATCGTGTGGGTGGAG, Primer 2 for UL8 fragment
· SEQ ID NO: 19:
   GTGTCGTGAGGCGCGCCATGGGGCAGGAAGACGGGAACCGCGGGGAG, Primer 1 for UL52 fragment
· SEQ ID NO: 20:
   GCCACCCGAAGAGCGGTACCTCAAGACGACGGTTGAGAGGTGCTGCAGGGAACGGACGGC, Primer 2 for UL52 fragment
· SEQ ID NO: 21:
   AGAGCTGAAGAGCCCTGCAGGATGACGGATTCCCCTGGCGGTGTGGCCCCC, Primer 1 for UL42 fragment
· SEQ ID NO: 22:
   ATGTATCTTAGAATTCTCAGGGGAATCCAAAACCATACGGGGTTTG, Primer 2 for UL42 fragment
· SEQ ID NO: 23:
   GAAGGCACAGTGTACATCACAGCATATCCAACGTCAGGTCTCCCTTTTTG, Primer 1 for UL29 fragment
· SEQ ID NO: 24:
   CAAAGAATTGGCTAGCATGGAGACAAAGCCCAAGACGGCAACCACC, Primer 2 for UL29 fragment
· SEQ ID NO: 25:
   GAAGGCACAGTGTACATCACAGCATATCCAACGTCAGGTCTCCCTTTTTG, Primer 1 for UL30 fragment
· SEQ ID NO: 26:
   CAAAGAATTGGCTAGCATGGAGACAAAGCCCAAGACGGCAACCACC, Primer 2 for UL30 fragment
· SEQ ID NO: 27:
   AGAGCTGAAGAGCCCTGCAGGATGTCGATGACGGCCTCGTCATCCACACCGCGGCC, Primer 1 for UL105 fragment
· SEQ ID NO: 28:
   ATGTATCTTAGAATTCTCAAAAAATAAGCGTGGTGCGTTTGTCTTTG, Primer 2 for UL105 fragment
· SEQ ID NO: 29:
   GAAGGCACAGTGTACATTAAGCGTTGAGCCGGAAAAACCGCAGGCC, Primer 1 for UL102 fragment
· SEQ ID NO: 30:
   CAAAGAATTGGCTAGCATGACCGCTCAGCCGCCGCTGCACCACCGC, Primer 2 for UL102 fragment
· SEQ ID NO: 31:
   GTGTCGTGAGGCGCGCCATGACGCTCGTTCTGTTCGCAACAGAGTATG, Primer 1 for UL70 fragment
· SEQ ID NO: 32:
   GCCACCCGAAGAGCGGTACCTCAGACGGCGGTCGCCGGCGGCATGGGCGC, Primer 2 for UL70 fragment
· SEQ ID NO: 33:
   AGAGCTGAAGAGCCCTGCAGGATGGATCGCAAGACGCGCCTCTCGGAGCCAC, Primer 1 for UL44 fragment
· SEQ ID NO: 34:
   ATGTATCTTAGAATTCCTAGCCGCACTTTTGCTTCTTGGTGTTAGG, Primer 2 for UL44 fragment
· SEQ ID NO: 35:
   GAAGGCACAGTGTACATTACAACCGGCTGCGTTTGGCCGGCAATAAC, Primer 1 for UL57 fragment
· SEQ ID NO: 36:
   CAAAGAATTGGCTAGCATGAGCCACGAGGAACTAACCGCGCTAGCG, Primer 2 for UL57 fragment
· SEQ ID NO: 37:
   GTGTCGTGAGGCGCGCCATGTTTTTCAACCCGTATCTGAGCGGCGGCGTGACCGG, Primer 1 for UL54 fragment
· SEQ ID NO: 38:
   GCCACCCGAAGAGCGGTACCTCAACAGCATTCGTGCGCCTTGACACTGTAC, Primer 2 for UL54 fragment
· SEQ ID NO: 39:
   CGAAAAGTGCCACCTGCGGCCGCAAGCTTTCTAGAACTAGTCCTAGGGGCTCCGGTGCCCG TCAGTG, Primer 1
· SEQ ID NO: 40:
   GCTGGCCTTTTGCTCGCGGCCGCAGATCTACGCGTGTCGA, Primer 2
· SEQ ID NO: 41:
   ATCCGAATTCGCTAGCATGGATCTGCCTACTACCGTCGTGCGAAAATAC, Primer 1 for UL112 fragment
· SEQ ID NO: 42:
   GCCACCCGAAGAGCGGTACCTTAATCGTCGAAAAACGCCGCGATCGAGGCGGCGGCGATC, Primer 2 for UL112 fragment
· SEQ ID NO: 43:
   TTAGTAAGCTTCCCTCCTGACGCGGTAGGAGGAGGGGAGGGTGCCCTGCATG, Primer 1 for VA fragment
· SEQ ID NO: 44:
   CCTTTTGCTCACATGTCAATTGGTAGATGTACCTGGACATCCAGGTGATGCCGGCG, Primer 2 for VA fragment
· SEQ ID NO: 45:
   TGTACAGGCGCGCCGAATTCGTTTTAGGGCGGAGTAACTTGTATGTGTTGGGAATTGTAG, Primer 1 for E4 fragment
· SEQ ID NO: 46:
   AGGGAAGCTTACTAAACGGTACACAGGAAACAGGAGACACAACTCCAAGTG, Primer 2 for E4 fragment
· SEQ ID NO: 47:
   GAAAAGTGCCACCTGACGTCGCGGCCGCGCGATCGCGGTACCCAACTCCATGCTCAACAGT CCCCAGGTACAGCCC, Primer 1 for E2A fragment
· SEQ ID NO: 48:
   GAATTCGGCGCGCCTGTACAGCCCGGGCGACCGCACCCTGTGACGAAAGCCGCCCGCAAG, Primer 2 for E2A fragment
· SEQ ID NO: 49:
   GAAAAGTGCCACCTGACGTCGCGGCCGCGGAGGGGTGGAGTCGTGACGTGAATTACGTCAT AGGGTTAGGGAGGTCCTGTATTAGAGGTCACGTGAGTGTTTTGCGAC, Primer 1 for p5 gene fragment
· SEQ ID NO: 50:
   GTTCAAACCTCCCGCTTCAAAATGGAGACCCTGCGTGCTCACTCGGGCTTAAATACCCAGC GTGACCACATGGTGTCGCAAAATGTCGCAAAACACTCA, Primer 2 for p5 gene fragment
· SEQ ID NO: 51:
   GCGGGAGGTTTGAACGCGCAGCCGCCATGCCGGGGTTTTAC, Primer 1 for AAV2 Rep fragment
· SEQ ID NO: 52:
   GATCGCAGCGCTATTTAAATCATTTATTGTTCAAAGAT, Primer 2 for AAV2 Rep fragment
· SEQ ID NO: 53: AATAGCGCTGCGATCGCGGCCTCCAAAAAAGC, Primer 1 for SV40 ori fragment
· SEQ ID NO: 54: CCTTTTGCTCACATGTCAATTGATCCCGCCCCTAACT, Primer 2 for SV40 ori fragment
· SEQ ID NO: 55: AACAATAAATGATTTAAATCAGG, Primer 1 for AAV1 Cap fragment
· SEQ ID NO: 56:
   GGCCGCGATCGCAGCGCTGTAGCCATGGAAACTAGATA, Primer 2 for AAV1 Cap fragment
· SEQ ID NO: 57: TCACCATCTTGTCGACACAGTCGTTGAAGGG, Primer 1 for p5/AAV2 Rep fragment
· SEQ ID NO: 58:
   AGCGCAGCGAGTCGGCCGGCCGCGGCCGCGGAGGGGTGGAGTCG, Primer 2 for p5/AAV2 Rep fragment
· SEQ ID NO: 59:
   ACATCTACGGATTACGTCGAggaggggtggagtcgtgacg, Primer 1 for p5/Cap fragment
· SEQ ID NO: 60:
   ACCGCATTAAAGCTCGTCGAAGCGCTgtagccatggaaac, Primer 2 for p5/Cap fragment
· SEQ ID NO: 61:
   gtgagcaaaaggGCGATCGCTCTAGAACTAGTgtagccatggaaactagata, Primer 1 for Rep2/Cap fragment
· SEQ ID NO: 62: CAACGACTGTGTCGACAAGATG, Primer 2 for Rep2/Cap fragment
· SEQ ID NO: 63:
   gaaaagtgccacctgcggccgcaagctttctaggcgttacataacttacggtaa, Primer 1 for CMV/hGH polyA fragment
· SEQ ID NO: 64:
   ggtaccGGCGCGCCggatccGCTCTTCagctctgcttatatagacctc, Primer 2 for CMV/hGH polyA fragment
· SEQ ID NO: 65:
   ggtaccGGCGCGCCggatccGCTCTTCagctctgcttatatagacctc, Primer 3 for CMV/hGH polyA fragment
· SEQ ID NO: 66:
   gctggccttttgctcgcggccgcagatctacgcgtgtcgacaaggacagggaagggagcag , Primer 4 for CMV/hGH polyA fragment
· SEQ ID NO: 67:
   gctGAAGAGCggatccgccaccatggacagctcccatctgcgcgatgttgtcatcaagctc c, Primer 1 for Ad5 polymerase fragment
· SEQ ID NO: 68:
   AGCggtaccGGCGCGCCtacggcatctcgatccagcatatctcctcgtttcgcgggttg, Primer 2 for Ad5 polymerase fragment

## Claims

1. A nucleic acid comprising an adenovirus-derived gene and a herpesvirus-derived gene.

2. The nucleic acid according to claim 1, wherein the herpesvirus-derived gene is a gene associated with replication of a gene.

3. The nucleic acid according to claim 1 or 2, wherein the herpesvirus-derived gene encodes a protein selected from a helicase, a primase, an ssDNA-binding protein (DBP), a DNA polymerase, and a DNA replication protein.

4. The nucleic acid according to any one of claims 1 to 3, wherein the herpesvirus-derived gene includes a gene selected from UL8, UL5, UL52, UL29 (ICP8), UL30, UL42, UL9, ORF6, ORF16, ORF28, ORF29, ORF55, BMRF1, BSLF1, BBLF4, BBLF2/BBLF3, BALF5, BALF2, UL102, UL105, UL70, UL57, UL54, UL44, UL112, U27, U38, U41, U79, U43, U73, U74, U77, U79, ORF9, and ORF59.

5. The nucleic acid according to any one of claims 1 to 3, wherein the herpesvirus-derived gene is selected from a DNA strand synthesis gene and a helicase-primase gene.

6. The nucleic acid according to claim 5, wherein the herpesvirus-derived gene includes a gene selected from UL8, UL5, UL52, UL29 (ICP8), UL30, UL42, UL102, UL105, UL70, UL57, UL54, UL44, and UL112.

7. The nucleic acid according to any one of claims 1 to 3, wherein the herpesvirus-derived gene includes a DNA strand synthesis gene.

8. The nucleic acid according to claim 7, wherein the herpesvirus-derived gene includes a gene selected from UL29 (ICP8), UL30, UL42, UL57, UL54, and UL44.

9. The nucleic acid according to any one of claims 1 to 3, wherein the herpesvirus-derived gene includes only one of a DNA strand synthesis gene or a helicase-primase gene.

10. The nucleic acid according to any one of claims 1 to 9, wherein the adenovirus-derived gene includes a helper gene.

11. The nucleic acid according to any one of claims 1 to 10, wherein the adenovirus-derived gene includes a gene selected from E1A, E1B, E2, E4, and VA.

12. The nucleic acid according to any one of claims 1 to 11, wherein the adenovirus is selected from serotypes 1 to 52.

13. The nucleic acid according to any one of claims 1 to 12, wherein the herpesvirus is selected from HHV-1 to HHV-8.

14. A composition for producing a viral vector, the composition comprising the nucleic acid according to any one of claims 1 to 13.

15. A composition for producing a virus-derived construct, the composition comprising a nucleic acid containing a gene associated with replication of a virus-derived gene.

16. The composition according to claim 15, wherein the gene associated with replication of the gene is derived from an adenovirus or a herpesvirus.

17. The composition according to claim 15 or 16, wherein the gene associated with replication of the gene is derived from a herpesvirus.

18. The composition according to any one of claims 15 to 17, wherein the gene associated with replication of the gene includes a gene selected from UL8, UL5, UL52, UL29 (ICP8), UL30, UL42, UL9, ORF6, ORF16, ORF28, ORF29, ORF55, BMRF1, BSLF1, BBLF4, BBLF2/BBLF3, BALF5, BALF2, UL102, UL105, UL70, UL57, UL54, UL44, UL112, U27, U38, U41, U79, U43, U73, U74, U77, U79, ORF9, and ORF59.

19. The composition according to any one of claims 15 to 17, wherein the gene associated with replication of the gene is selected from a DNA strand synthesis gene and a helicase-primase gene.

20. The composition according to claim 19, wherein the gene associated with replication of the gene includes a gene selected from UL8, UL5, UL52, UL29 (ICP8), UL30, UL42, UL102, UL105, UL70, UL57, UL54, UL44, and UL112.

21. The composition according to any one of claims 15 to 17, wherein the gene associated with replication of the gene includes a DNA strand synthesis gene.

22. The composition according to claim 21, wherein the gene associated with replication of the gene includes a gene selected from UL29 (ICP8), UL30, UL42, UL57, UL54, and UL44.

23. The composition according to any one of claims 15 to 17, wherein the gene associated with replication of the gene includes only one of a DNA strand synthesis gene or a helicase-primase gene.

24. The composition according to any one of claims 15 to 23, wherein the gene associated with replication of the gene is derived from a herpesvirus selected from HHV-1 to HHV-8.

25. The composition according to any one of claims 15 to 24, wherein the gene associated with replication of the gene is derived from an adenovirus selected from serotypes 1 to 52.

26. The composition according to any one of claims 15 to 25, wherein the composition is for producing an adeno-associated virus (AAV) vector.

27. The nucleic acid according to any one of claims 1 to 13, wherein the nucleic acid is a plasmid.

28. The nucleic acid according to any one of claims 1 to 13 or 27, wherein the nucleic acid forms a complex with a cationic substance.

29. A method for producing a producer cell that produces a virus-derived construct, the method comprising a step of introducing the nucleic acid according to any one of claims 1 to 23, 27, or 28 into a producer cell.

30. The method according to claim 29, wherein at least a part of the nucleic acid is integrated into a chromosome of the producer cell.

31. A producer cell produced by the method according to claim 29.

32. A producer cell comprising the nucleic acid according to any one of claims 1 to 13, 27, or 28.

33. A method for producing a virus-derived construct, the method comprising:
a step of introducing the nucleic acid according to any one of claims 1 to 13, 27, or 28 into a producer cell; and
a step of forming a virus-derived construct in the producer cell.

34. A virus-derived construct produced by the method according to claim 33.

35. A virus-derived construct comprising the nucleic acid according to any one of claims 1 to 13, 27, or 28.
